# EUROPEAN PATENT SPECIFICATION

(11) **EP 3 473 728 B1**
(45) Date of publication and mention of the grant of the patent: **20.10.2021**
(21) Application number: 17813591.9
(22) Date of filing: 14.06.2017
(51) Int. Cl.: C12Q 1/44, C12N 15/10, C12N 15/113, C12N 15/65, C12N 15/90, C12N 9/22

(54) **METHOD FOR SCREENING TARGETED GENETIC SCISSORS BY USING MULTIPLE TARGET SYSTEM OF ON-TARGET AND OFF-TARGET ACTIVITY AND USE THEREOF**
VERFAHREN ZUM SCREENING VON GEZIELTEN GENETISCHEN SCHEREN UNTER VERWENDUNG EINES MEHRFACHZIELSYSTEMS MIT ON-TARGET- UND OFF-TARGET-AKTIVITÄT UND VERWENDUNG DAVON
PROCÉDÉ DE CRIBLAGE DE CISEAUX GÉNÉTIQUES CIBLÉS À L'AIDE D'UN SYSTÈME À CIBLES MULTIPLES D'ACTIVITÉ SUR CIBLE ET HORS CIBLE ET SON UTILISATION

(30) Priority: 15.06.2016 US 201662350282 P
(43) Date of publication of application: 24.04.2019
(73) Proprietor: Toolgen Incorporated, Seoul 08501 (KR)
(72) Inventor: LEE, Jeong Joon, Seoul 03093 (KR)
(74) Representative: dompatent von Kreisler Selting Werner - Partnerschaft von Patent- und Rechtsanwälten mbB
(86) International application number: PCT/KR2017/006212
(87) International publication number: WO 2017/217768

(56) References cited:
- KR-A- 20150 101 446
- KR-A- 20150 138 074
- MARK J OSBORN ET AL: "Evaluation of TCR Gene Editing Achieved by TALENs, CRISPR/Cas9, and megaTAL Nucleases", MOLECULAR THERAPY : THE JOURNAL OF THE AMERICAN SOCIETY OF GENE THERAPY, vol. 24, no. 3, 5 January 2016 (2016-01-05), pages 570-581, XP055278002, US ISSN: 1525-0016, DOI: 10.1038/mt.2015.197
- MARIANNA PAULIS ET AL: "A pre-screening FISH-based method to detect CRISPR/Cas9 off-targets in mouse embryonic stem cells", SCIENTIFIC REPORTS, vol. 5, no. 1, 24 July 2015 (2015-07-24), XP055461982, DOI: 10.1038/srep12327
- DAESIK KIM ET AL: "Digenome-seq: genome-wide profiling of CRISPR-Cas9 off-target effects in human cells", NATURE METHODS, vol. 12, no. 3, 1 March 2015 (2015-03-01), pages 237-243, XP055554961, New York ISSN: 1548-7091, DOI: 10.1038/nmeth.3284
- XIAOLING WANG ET AL: "Unbiased detection of off-target cleavage by CRISPR-Cas9 and TALENs using integrase-defective lentiviral vectors", NATURE BIOTECHNOLOGY, vol. 33, no. 2, 19 January 2015 (2015-01-19), pages 175-178, XP055548847, New York ISSN: 1087-0156, DOI: 10.1038/nbt.3127
- RICHARD GABRIEL ET AL: "An unbiased genome-wide analysis of zinc-finger nuclease specificity", NATURE BIOTECHNOLOGY, vol. 29, no. 9, 1 January 2011 (2011-01-01), pages 816-823, XP055073828, ISSN: 1087-0156, DOI: 10.1038/nbt.1948
- LAUGHERY, MARIAN F. et al.: "New Vectors for Simple and Streamlined CRISPR-Cas9 Genome Editing in Saccharomyces Cerevisiae", Yeast, vol. 32, no. 12, 2015, pages 71 1-720, XP055449318,
- HENDEL, AYAL et al.: "Quantifying On- and Off-target Genome Editing", Trends in Biotechnology, vol. 33, no. 2, February 2015 (2015-02), pages 132-140, XP055449327,
- LEE, CIARANM. et al.: "Nuclease Target Site Selection for Maximizing On-target Activity and Minimizing Off-target Effects in Genome Editing", Molecular Therapy, vol. 24, no. 3, March 2016 (2016-03), pages 475-487, XP055449327,

## Description

### [Technical Field]

The present invention relates to a method for selecting a target specific nuclease having high specificity and high activity and, more particularly, to a method for screening and selecting a target specific nuclease system by using a multiple target system capable of simultaneously identifying off-target activity and on-target activity.

### [Background Art]

A CRISPR-Cas system consists of a guide RNA having a sequence complementary to a gene or nucleic acid to be targeted and a CRISPR enzyme which is an enzyme cleaving the gene or nucleic acid to be targeted, and the guide RNA and the CRISPR enzyme form a CRISPR complex and cleave or modify a gene or nucleic acid targeted by the formed CRISPR complex.

However, a problem of modifying a gene or nucleic acid that is not targeted along with the aforementioned effect of modifying the gene or nucleic acid to be targeted still has not been solved.

The gene or nucleic acid that is not targeted can partially interact with the guide RNA by including a sequence partially complementary to the guide RNA, and the CRISPR complex cleaves or modifies the corresponding gene, that is, an undesired gene or nucleic acid, by the interaction. This effect is referred to as an off-target effect.

Accordingly, it is important to increase an on-target effect in order to increase the efficiency of modifying the targeted gene or nucleic acid by using the CRISPR-Cas system, or to reduce or remove an off-target effect of the CRISPR-Cas system in order to solve a problem such as genetic defects which may be caused by modification of an undesired gene or nucleic acid. Further, it is possible to expect both the accuracy and specificity with which a gene or nucleic acid to be targeted can be exactly modified as the off-target effect of the aforementioned CRISPR-Cas system is reduced.

In order to reduce or remove the off-target effect of the CRISPR-Cas system and/or improve the accuracy and specificity of the CRISPR-Cas system, it is considered as an important part to select (that is, to select a target having a small off-target effect) or modify a guide RNA having a small off-target effect and regulate the activity (or efficiency) and specificity of the CRISPR enzyme.

In order to regulate the activity or specificity of the CRISPR enzyme, various strategies such as a method for regulating the concentration of Cas9 in cells or causing a single stranded DNA break using a Cas9 nickase, a method for using a fusion protein produced by fusing a FokI nuclease domain with catalytically inactivated Cas9, and the use of a truncated guide RNA in which partial sequences of the 5' end of the guide RNA are removed have been attempted and developed.

Therefore, the present inventors developed a screening method for selecting a target specific nuclease having high specificity and/or activity, and confirmed that an excellent target specific nuclease could be effectively selected among candidate groups of various modified nucleases by using the screening method, thereby completing the present invention.

### [Prior Art Document]

Patent Documents
WO 2013-098244
WO 2015-123339
WO 2014-093661
WO 2014-204724
KR 10-2016-0058703
Non-Patent Documents

1. Anders, C. et al., 2014. Structural basis of PAM-dependent target DNA recognition by the Cas9 endonuclease. Nature 513 (7519), 569-573.
2. Anderson, E.M., et al., 2015. Systematic analysis of CRISPR-Cas9 mismatch tolerance reveals low levels of off-target activity. J. Biotechnol. 211, 56-65.
3. Frock, R.L.et al., 2015. Genome-wide detection of DNA double-stranded breaks induced by engineered nucleases. Nat. Biotechnol. 33(2), 179-186
4. Bauer, D.E. et al., 2015. Generation of genomic deletions in mammalian cell lines via CRISPR/Cas9. J. Vis. Exp. 95.
5. Brinkman, E.K. et al., 2014. Easy quantitative assessment of genome editing by sequence trace decomposition. Nucleic Acids Res. 42(22), e168.
6. Cho, S.W.et al., 2014. Analysis of off-target effects of CRISPR/Cas-derived RNA-guided endonucleases and nickases. Genome Res. 24 (1), 132-141.
7. Christian, M.et al. 2010. Targeting DNA double-strand breaks with TAL effector nucleases. Genetics 186 (2), 757-761.
8. Cradick, T.J.et al., 2013. CRISPR/Cas9 systems targeting β-globin and CCR5 genes have substantial off-target activity. Nucleic Acids Res. 41 (20), 9584-9592.
9. Crosetto, N.et al., 2013. Nucleotide-resolution DNA double-strand break mapping by next-generation sequencing. Nat. Methods 10 (4), 361-365.
10. Dahlem, T.J.et al., 2012. Simple methods for generating and detecting locus-specific mutations induced with TALENs in the zebrafish genome. PLoS Genet. 8 (8), e1002861.
11. Doench, J.G.et al., 2016. Optimized sgRNA design to maximize activity and minimize off-target effects of CRISPR-Cas9. Nat. Biotechnol. 34 (2), 184-191
12. Fu, Y.et al., 2013a. High frequency off-target mutagenesis induced by CRISPR-Cas nucleases in human cells. Nat. Biotechnol. 31 (9), 822-826
13. Osborn, M. J., et al., 2016, Evaluation of TCR Gene Editing Achieved by TALENs, CRISPR/Cas9, and megaTAL Nucleases. Molecular Therapy: The Journal of the American Society of Gene Therapy, 24 (3), 570-581
14. Paulis, M., et al., 2015, A pre-screening FISH-based method to detect CRISPR/Cas9 off-targets in mouse embryonic stem cells. Scientific Reports, 5 (1)
15. Kim, D., et al., 2015, Digenome-seq: genome wide profiling of CRISPR-Cas9 off-targets effects in human cells, Nature Methods 12 (3), 237-243
16. Wang, X., et al., 2015, Unbiased detection of off-target cleavage by CRISPR-Cas9 and TALENs using integrase-defective lentiviral vectors, Nature Biotechnology, 33 (2), 175-178
17. Gabriel, R., et al., 2011, An unbiased genome-wide analysis of zinc-finger nuclease specificity, Nature Biotechnology, 29(9), 816-823

### [Disclosure]

### [Technical Problem]

An object of the present invention is to provide a screening system for selecting a target specific nuclease using multiple targets including one or more on-targets and one or more off-targets.

Another object of the present invention is to provide a selection system of a target specific nuclease using multiple targets including one or more on-targets and one or more off-targets.

Still another object of the present invention is to provide a kit or composition for screening and/or selecting a target specific nuclease using multiple targets.

Yet another object of the present invention is to provide a cell including multiple targets for screening and/or selecting a target specific nuclease and/or a method for producing the cell.

Still yet another object of the present invention is to provide a system for screening and selecting a target specific nuclease using multiple targets, and all the uses of the selected nuclease by using the same.

### [Technical Solution]

To solve the problems, the present invention provides a screening system for selecting a target specific nuclease having high specificity or high activity, and various uses of the same according to claims 1, 6, 8, 13 and 15. Preferred embodiments are claimed in the dependent claims.

The target specific nuclease may include a part capable of specifically recognizing a target and a part capable of cleaving or modifying the recognized -target, and can screen and/or select a part capable of specifically recognizing a target and/or a part capable of cleaving or modifying the recognized target through the screening system.

### Screening System

As used herein, the term "screening system for selecting a target specific nuclease" refers to a concept including all of a composition and a kit used to select a target specific nuclease having high specificity and high activity, a method for using the same, and various intermediates derived during the processes.

Therefore, the invention described as a "system" in the present specification may be interpreted as a composition or method so as to be suitable for the aspects of the corresponding inventions as long as the present invention is used for selecting a desired target specific nuclease.

An aspect of the present invention relates to a method for screening a target specific nuclease system and a composition used for the same.

Another aspect of the present invention relates to a method for selecting a target specific nuclease having high specificity or high activity through the screening method and a composition used for the same.

As a preferred specific embodiment, the present invention provides
a screening system or composition for selecting a target specific nuclease having high specificity or high activity, the screening system or composition including:
i) a nuclease including a part capable of recognizing a target and a part capable of cleaving or modifying the recognized target; and
ii) multiple targets including one or more selection elements, in which the multiple targets include one or more on-targets and one or more off-targets.

As another preferred specific embodiment, the present invention provides
a screening system or composition for selecting a target specific nuclease having high specificity or high activity, the screening system or composition including:
i) a nuclease including a part capable of recognizing a target and a part capable of cleaving or modifying the recognized target; and
ii) multiple targets including one or more selection elements, in which the multiple targets include one on-target and one or more off-targets.

As still another preferred specific embodiment, the present invention provides
a screening system or composition for selecting a target specific nuclease having high specificity or high activity, the screening system or composition including:
i) a nuclease including a part capable of recognizing a target and a part capable of cleaving or modifying the recognized target; and
ii) multiple targets including one selection element, in which the multiple targets include one on-target and one off-target.

The screening system may be used to select an excellent target specific nuclease having high specificity and/or high activity among various target candidate groups.

### Nuclease

The term "target specific nuclease" refers to a nuclease capable of recognizing and cleaving a specific position of a nucleic acid (DNA or RNA) in a desired (target or targeted) genome. In the present invention, the target specific nuclease is also used as a concept including other elements required to enable a specific nuclease to perform a desired function.

The nuclease may include a nuclease in which a domain (part) and a domain (part) capable of recognizing and cleaving a specific target sequence in a genome, respectively, are fused, and examples thereof may include a meganuclease, a fusion protein in which a transcription activator-like (TAL) effector domain and a cleavage domain are fused which is a transcription activator-like effector nuclease (TALEN) derived from a plant pathogenic gene which is a domain capable of recognizing a specific target sequence in a genome, a zinc-finger nuclease, or a RNA-guided engineered nuclease (RGEN) without limitation. In the present invention, the RGEN is used.

In the present invention, the "RGEN" includes a CRISPR-Cas system consisting of a target-specific guide RNA and a CRISPR enzyme.

The "CRISPR-Cas system" of the present invention includes sequences encoding the guide RNA and the CRISPR enzyme, and includes all the elements involved in the induction of the expression or activity thereof. The CRISPR-Cas system may be a vector system including sequences encoding the guide RNA and the CRISPR enzyme.

The constituent elements included in the vector system are operably linked to each other. The term "operably linked" refers to a functional connection between nucleic acid sequences such that the sequence encodes a desired function. For example, a desired gene, for example, a coding sequence for a selectable marker, when a linked promoter and/or a regulatory region functionally regulate/regulates the expression of the coding sequence, is in an operably linked state with a promoter thereof and/or a regulatory sequence thereof. This refers to a connection between coding sequences such that these can also be regulated by the same linked promoter and/or regulatory region, and this connection between coding sequences may also refer to a connection at a frame or the same coding frame. The term "operably linked" also refers to a connection of a functional, but non-coding sequence, for example, a self-proliferation sequence or a replication origin. These sequences are in an operably linked state when these can perform normal functions thereof, for example, when these can replicate, proliferate, and/or separate a vector including the sequence in a host cell.

In addition, the "CRISPR-Cas system" of the present invention includes a sequence of a guide RNA and a protein or polypeptide of a CRISPR enzyme, and includes all the elements involved in the induction of the expression or activity thereof, and the CRISPR-Cas system may be a ribonucleoprotein (RNP) system in which the guide RNA and the CRISPR enzyme form a complex. In this case, the RNP is a protein-RNA complex, and refers to a form of a complex formed by the interaction of RNA and an RNA-binding protein.

The "CRISPR enzyme" of the present invention is a main protein component of the CRISPR-Cas system, and forms a complex with a guide RNA, thereby forming an activated CRISPR-Cas system or an inactivated CRISPR-Cas system.

In this case, the inactivated CRISPR-Cas system refers to a CRISPR-Cas system including a CRISPR enzyme in which all or a part of the functions of the CRISPR enzyme are inactivated, and the CRISPR-Cas system with the functions partially inactivated may serve as a nickase that cleaves a single-stranded nucleic acid.

As an example, when the CRISPR enzyme is Cas9, the CRISPR enzyme may be prepared by introducing the mutation of D10A or H840A into a Cas9 protein, and a D10A Cas9 protein, an H840A Cas9 protein, that is, a Cas9 protein produced by introducing a mutation into only one active site of the Cas9 protein may serve as a nickase when binding to the guide RNA.

As another example, the D10A/H10A Cas9 protein produced by introducing the mutations of both D10A and H840A into the Cas9 protein, that is, an inactivated Cas9 protein (dead Cas9, dCas9) produced by introducing the mutation into two active sites of the Cas9 protein may serve as a nucleic acid binding complex which does not cleave a target gene or a nucleic acid sequence when the inactivated Cas9 protein binds to a guide RNA.

The CRISPR enzyme is a nucleic acid having a sequence encoding a CRISPR enzyme or polypeptide (or protein)and representatively, a Type II CRISPR enzyme or Type V CRISPR enzyme is usually used.

Examples of the Type II CRISPR enzyme include Cas9, and the Cas9 may be Cas9 derived from various microorganisms, such as Actinobacteria (for example, *Actinomyces naeslundii)* Cas9, Aquificae Cas9, Bacteroidetes Cas 9, Chlamydiae Cas9, Chloroflexi Cas9, Cyanobacteria Cas9, Elusimicrobia Cas9, Fibrobacteres Cas9, Firmicutes Cas9 (for example, *Streptococcus pyogenes* Cas9, *Streptococcus thermophilus* Cas9, *Listeria innocua* Cas9, *Streptococcus agalactiae* Cas9, *Streptococcus mutans* Cas9, and *Enterococcus faecium* Cas9), Fusobacteria Cas9, Proteobacteria (for example, *Neisseria meningitides, Campylobacter jejuni)* Cas9, and Spirochaetes (for example, *Treponema denticola*) Cas9.

The "Cas9" is an enzyme capable of cleaving or modifying a sequence or position of a gene or nucleic acid to be targeted by binding to a guide RNA, and may consist of an HNH domain capable of cleaving a nucleic acid strain complementarily binding to the guide RNA, a RuvC domain capable of cleaving a nucleic acid strand non-complementarily binding to the guide RNA, a REC domain capable of recognizing a target, and a PI domain capable of recognizing PAM For specific structural characteristics of Cas9, Hiroshi Nishimasu et al. (2014) Cell 156:935-949 may be referenced.

In addition, examples of the Type V CRISPR enzyme include Cpfl, and the Cpfl may be Cpfl derived from Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium, Corynebacter, Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Letospira, Desulfovibrio, Desulfonatronum, Opitutaceae, Tuberibacillus, Bacillus, Brevibacilus, Methylobacterium, or Acidaminococcus.

Examples of the Cpfl include a similar RuvC domain corresponding to a RuvC domain of Cas9, the HNH domain of Cas9 is deficient, a Nuc domain is included instead, and the Cpfl may consist of a REC domain and a WED domain capable of recognizing a target and a PI domain capable of recognizing PAM. For specific structural characteristics of Cpfl, Takashi Yamano et al. (2016) Cell 165:949-962 may be referenced.

The CRISPR enzyme can recognize a protospacer adjacent motif (PAM) in a gene or nucleic acid sequence, and the PAM may vary according to the type and/or source of CRISPR enzyme.

For example, the PAM may be 5'-NGG-3' when the CRISPR enzyme is SpCas9, the PAM may be 5'-NNAGAAW-3' (W = A or T) when the CRISPR enzyme is StCas9, the PAM may be 5'-NNNNGATT-3' when the CRISPR enzyme is NmCas9, the PAM may be 5'-NNNVRYAC-3' (V = G or C or A, R = A or G, and Y = C or T) when the CRISPR enzyme is CjCas9, and in this case, the N may be A, T, G or C; or A, U, G or C. Furthermore, the PAM may be 5' TTN-3' when the CRISPR enzyme is FnCpfl, the PAM may be 5'-TTTN-3' when the CRISPR enzyme is AsCpfl or LbCpfl, and in this case, the N may be A, T, G or C; or A, U, G or C.

The information of the CRISPR enzyme can be obtained from a publicly-known database such as the GenBank of the National Center for Biotechnology Information (NCBI), but the source is not limited thereto.

As used herein, the term "guide RNA" refers to a target gene or nucleic acid specific RNA, and the guide RNA can direct the CRISPR enzyme to a target gene or nucleic acid by binding to the CRISPR enzyme.

The guide RNA may consist of a crRNA including a sequence complementary to a gene or nucleic acid sequence to be targeted and a tracrRNA binding to a CRISPR enzyme, and in this case, the crRNA includes a guide sequence which is a part capable of complementarily binding to a gene or nucleic acid sequence to be targeted.

The guide sequence has a sequence complementary to a gene or nucleic acid sequence to be targeted, and serves to recognize a gene or nucleic acid sequence to be targeted. Further, the crRNA includes a part having a sequence complementary to a portion of a tracrRNA, and thus may partially complementarily bind to the tracrRNA.

In this case, the guide RNA may be a dual guide RNA in which the crRNA and the tracrRNA are each separately present, or a single guide RNA in which the crRNA and the tracrRNA are connected to each other, and may include a linker when the guide RNA is a single guide RNA. The guide RNA may include only a crRNA according to the type of CRISPR enzyme.

In another embodiment of the present disclosure, the target specific nuclease may be a zinc-finger nuclease (ZFN).

The ZFN includes a zinc-finger protein engineered so as to bind to a selected gene and a target site in a cleavage domain or a cleavage half-domain. A zinc-finger binding domain may be engineered so as to bind to a selected sequence. For example, Beerli et al. (2002) Nature Biotechnol. 20:135-141; Pabo et al. (2001) Ann. Rev. Biochem. 70:313-340; Isalan et al. (2001) Nature Biotechnol. 19: 656-660; Segal et al. (2001) Curr. Opin. Biotechnol. 12:632-637; Choo et al. (2000) Curr. Opin. Struct. Biol. 10:411-416 can be referenced. When compared with a naturally occurring zinc finger protein, the engineered zinc finger binding domain may have novel binding specificity. The engineering method includes a rational design and various types of selections, but is not limited thereto. The rational design includes the use of a database including, for example, a triple (or quadruple) nucleotide sequence, and individual zinc finger amino acid sequences, and in this case, the respective triple or quadruple nucleotide sequence is combined with one or more sequences of zinc fingers which bind to a certain triple or quadruple sequence.

The selection of a target sequence and the design and construction of a fusion protein (and a polynucleotide encoding the fusion protein) are known to those skilled in the art, and are described in detail in U.S. Patent Publication Nos. 2005/0064474 and 2006/0188987, the entire contents of which are incorporated herein by reference.

Further, as disclosed in these references and the other references, zinc finger domains and/or multiple-finger zinc finger proteins can be linked together by any suitable linker sequences, for example, a linker including five or more amino acids in length. For examples of the linker sequence of six or more amino acids in length, U.S. Patent Nos. 6,479,626; 6,903,185; and 7,153,949 can be referenced. The proteins described herein may include any combination of suitable linkers between each of the zinc fingers of the proteins.

In addition, a target specific nuclease such as ZFN and/or a meganuclease may include a cleavage domain or a cleavage half-domain.

As has been well known, for example, as in a cleavage domain from a nuclease different from a zinc finger DNA binding domain or a cleavage domain from a nuclease different from a meganuclease DNA binding domain, the cleavage domain may be heterologous to the DNA binding domain. The heterologous cleavage domain can be obtained from any endonuclease or exonuclease. An exemplary endonuclease from which the cleavage domain can be derived includes a restriction endonuclease and a meganuclease, but is not limited thereto.

The restriction endonucleases (restriction enzymes) are present in many species, and can be sequence-specifically bound to DNA (at a target site), thus cleaving DNA at or near the binding site. Some restriction enzymes (for example, Type IIS) cleave DNA at a site removed from the recognition site, and have separable binding and cleavable domains. For example, the type IIS enzyme Fokl catalyzes a double-stranded cleavage of DNA at nine nucleotides from the recognition site on one strand and at 13 nucleotides from the recognition site on the other strand. Accordingly, in one embodiment, the fusion protein includes at least one cleavage domain from a Type IIS restriction enzyme and one or more zinc-finger domains (that may or may not be engineered).

As used herein, the term "TALE domain" refers to a protein domain capable of binding to a nucleotide in a sequence-specific manner through one or more TALE-repeating modules. The TALE domain includes at least one TALE-repeating module, preferably, 1 to 30 TALE-repeating modules, but is not limited thereto. In the present invention, the terms "TAL effector domain" and "TALE domain" can be interchangeably used. The TALE domain may include half of the TALE-repeating modules.

### Multiple Targets

The present invention is characterized by using multiple targets system or simultaneous targets system capable of simultaneously identifying an on-target effect (activity) and/or an off-target effect (activity).

The "target" of the present invention is an object to be cleaved or modified by a target specific nuclease, and can be interchangeably used with a target or desired gene or nucleic acid or a sequence thereof.

The part capable of recognizing a "target" of a target specific nuclease includes a nucleic acid sequence complementary to a target gene or nucleic acid sequence. The nucleic acid may be RNA, DNA or a RNA/DNA hybrid, but is not limited thereto.

As used herein, the term "multiple targets" is a concept including one or more on-target effects and one or more off-target effects, and refers to targets that form a system capable of simultaneously or individually identifying an on-target effect and an off-target effect of a test nuclease.

Therefore, the "multiple targets" may be a gene or nucleic acid sequence targeted by a target specific nuclease, the multiple targets may be two or more targets, and the multiple targets may consist of an on-target and an off-target.

The on-target refers to a sequence or position of a target gene or nucleic acid to which a target specific nuclease complementarily binds, and the off-target refers to a sequence or position of a target gene or nucleic acid to which a target specific nuclease partially complementarily binds and where the activity of the nuclease, which is not desired, occurs. The off-target may be a sequence or position of a gene or nucleic acid that the target specific nuclease does not target; or a nucleic acid sequence having a sequence homology less than 100% with the nucleic acid sequence of the on-target. The nucleic acid sequence having a sequence homology less than 100% with the nucleic acid sequence of the on-target is a nucleic acid sequence similar to the nucleic acid sequence of the on-target, and may be a nucleic acid sequence in which one or more different base sequences are included or one or more base sequences are deleted.

The multiple targets may consist of one or more on-targets and one or more off-targets, the number of each of the on-targets and the off-targets is not limited, and further, the numbers of on-targets and off-targets may not be the same. That is, the multiple targets may be multiple targets including one on-target and two off-targets, and in addition, the multiple targets may be multiple targets including two on-targets and five off-targets, and the multiple targets can be designed without being limited thereto.

Furthermore, in order to increase the reliability of selection of the screening system of the present invention, an object to be cleaved or modified by a target specific nuclease can be designed as a plurality of targets, desired genes, nucleic acids, or sequences thereof. The plurality of targets means that the number of targets is two or more, the number of targets may be 2, 3, 4, 5, 6, 7, or more, and the number thereof is not limited.

In this case, the configuration of a system can be designed in consideration of the plurality of targets and the multiple targets.

The multiple targets are connected to a selection element capable of identifying on-target and off-target effects.

### Selection Element

The term "selection element" of the present invention refers to a marker for detecting whether a target gene or nucleic acid sequence is cleaved or modified by a target specific nuclease. Therefore, the "selection element" can be used interchangeably with "a selection marker".

Based on whether the selection element is expressed or not, it is possible to detect whether a target gene or nucleic acid sequence is cleaved or modified by a target specific nuclease. That is, it is possible to detect whether the on-target effect is increased and/or whether the off-target effect is decreased.

The selection element is a toxin gene, an antibiotic resistance gene, a gene encoding fluorescent protein, a tagging gene, a lacZ gene or a gene encoding luciferase, and is not limited thereto.

The "toxin gene" is a gene or nucleic acid sequence encoding a toxic material (protein), the toxic material is produced by expression of the toxin gene, and it is possible to regulate the viability of a cell including the toxin gene by the produced toxic material. Alternatively, the toxin gene may be a material capable of regulating the viability of a cell by affecting the synthesis or breakdown of a material required for the viability of the cell including the toxin gene.

The toxin gene may be Hok, fst, TisB, LdrD, FlmA, Ibs, TxpA/BrnT, SymE, XCV1262, CcdB, ParE, MazF, yafO, HicA, Kid, Zeta, DarT, the Sxt gene, the hypoxanthine phosphoribosyl transferase (HPRT) gene, or URA3, and is not limited thereto.

When a host is *E. coli*(*Escherichia coli),* the toxin gene ccdB can be used.

When a host cell is a mammalian cell, the toxin gene HPRT can be used.

When the host is yeast, the toxin gene URA3 can be used.

The "antibiotic resistance gene" may be a gene or nucleic acid sequence which allows an antibiotic resistance trait to be expressed in a state of exposure to antibiotics.

The antibiotic may be kanamycin, spectinomycin, streptomycin, ampicillin, carbenicillin, bleomycin, erythromycin, polymyxin B, tetracycline, zeocin, puromycin, or chloramphenicol, and is not limited thereto.

The "fluorescent protein" is a protein expressing fluorescence, and may be a green fluorescent protein (GFP), a red fluorescent protein (RFP), a yellow fluorescent protein (YFP), a cyan fluorescent protein (CFP), a blue fluorescent protein (BFP), or an orange fluorescent protein (OFP), and is not limited thereto.

The tag may be AviTag, Calmodulin-tag, polyglutamate tag, E-tag, FLAG-tag, HA-tag, His-tag, Myc-tag, NE-tag, S-tag, SBP-tag, Softag 1, Softag 3, Strep-tag, TC tag, V5 tag, VSV-tag, Xpress tag, Isopeptag, SpyTag, SnoopTag, Biotin Carboxyl Carrier Protein(BCCP), Glutathione-S-transferase(GST)-tag, HaloTag, Maltose binding protein(MBP)-tag, Nus-tag, Thioredoxin-tag, chitin binding protein(CBP), thioredoxin(TRX), or poly(NANP). Further, the tag can use a fluorescent protein.

As a specific embodiment of the present invention, one or more selection elements can be used as the selection element for identifying the on-target effect and the off-target effect by the nuclease.

As an example, when a lacZ gene is used as the selection element, lacZ is not expressed if a target gene or nucleic acid sequence including lacZ is cleaved or modified by a target specific nuclease, which can be detected by a blue white screen method. The blue white screen method is a screening method using a β-galactosidase expressed from a lacZ gene, and when colorless 5-bromo-4-chloro-3-indolyl β-D-galactopyranoside (X-gal) is cleaved by the β-galactosidase, 5-bromo-4-chloro-indoxyl is formed and spontaneously becomes a dimer, thereby forming 5,5'-dibromo-4,4'-dichloro-indigo which is an insoluble pigment which is oxidized to exhibit a bright color. Accordingly, it is possible to identify whether a lacZ gene is expressed or not by using a phenomenon in which β-galactosidase-expressing cells exhibit a blue color in the presence of X-gal, and it is possible to predict the cleavage or modification of a target gene or nucleic acid sequence including a lacZ gene by using the same.

As another example, when a target specific nuclease is screened by using an on-target including a toxin gene as a selection element and an off-target present in a genome of a cell, if a vector including the on-target and a vector including the target specific nuclease are introduced into a cell, it is possible to expect that the target specific nuclease exhibiting high specificity cleaves the on-target, and as a result, the expression of the toxin gene is suppressed, whereas the off-target is not cleaved, and as a result, the genome is not cleaved. Therefore, a cell where only the on-target is cleaved and the off-target is not cleaved, into which a target specific nuclease having high specificity is introduced, can survive, and a cell in which both the on-target and the off-target are cleaved cannot survive because the genome is cleaved due to the cleavage of the off-target, so that the two types of cells can be distinguished from each other.

As still another example, when a target specific nuclease is screened by using an on-target including a toxin gene as a selection element and an off-target including a gene encoding fluorescent protein as a selection element, if a vector including the on-target and a vector including the target specific nuclease are introduced into a cell, it is possible to expect that the target specific nuclease exhibiting high specificity cleaves the on-target, and as a result, the expression of the toxin gene is suppressed, whereas the off-target is not cleaved, and as a result, the fluorescent protein is expressed. Therefore, a cell where only the on-target is cleaved and the off-target is not cleaved, into which a target specific nuclease having high specificity is introduced, can survive, and can also be distinguished with a fluorescent protein.

Accordingly, it is possible to select a target specific nuclease having high specificity to effectively cleave only an on-target based on whether these selection elements are expressed or not.

### Selection of Nuclease

A cell including a target specific nuclease having high specificity or high activity is sorted from test nucleases by using the screening system, and a desired nuclease can be selected (identified) therefrom.

As a specific embodiment, the present invention provides a method for selecting a target specific nuclease, the method including:
a) introducing i) and ii) components of the screening system into a cell;
b) sorting the cell by identifying the on-target effect and the off-target effect based on whether a selection element is expressed or not in the a); and
c) selecting the nuclease present in the sorted cell in the b) as the target specific nuclease having high specificity or high activity.

For the introducing of i) and ii) components into the cell, a publicly-known method can be used.

In some specific embodiments, the cell is transfected. A suitable transfection method includes calcium phosphate-mediated transfection, nucleofection, electroporation, cationic polymer transfection (for example, DEAE-dextran or polyethyleneimine), virus transfection, virosome transfection, virion transfection, liposome transfection, cationic liposome transfection, immunoliposome transfection, non-liposome transfection, dendrimer transfection, heat shock transfection, magnetofection, lipofection, gene gun delivery, impalefection, sonoporation, optical transfection, and proprietary agent-enhanced uptake of nucleic acids. Transfection methods are widely known in the art (see, for example, "Current Protocols in Molecular Biology" Ausubel et al., John Wiley & Sons, New York, 2003 or "Molecular Cloning: A Laboratory Manual" Sambrook & Russell, Cold Spring Harbor Press, Cold Spring Harbor, NY, 3rd edition, 2001). In a preferred embodiment, i) and ii) components can be introduced into the cell by electroporation.

In a specific embodiment, a DNA encoding i) or ii) component may be present in a vector. A suitable vector includes a plasmid vector, a phagemid, a cosmid, artificial/kid chromosomes, a transposon, and a virus vector (for example, a lentivirus vector, an adeno-associated virus vector, and the like).

As used herein, the term "vector" refers to a carrier nucleic acid molecule utilized to transfer a desired (intended, specific, or necessary) nucleic acid, the vector may be a single-stranded nucleic acid, a double-stranded nucleic acid or a partial double-stranded nucleic acid, and further, the vector may consist of one or more free ends or no-free ends (e.g. circular).

A "promoter" can be included as a regulatory element that regulates the expression of a gene in a vector. The promoter may vary according to the cell into which a vector is introduced. The promoter may be a pol III promoter (for example, a U6 promoter, an H1 promoter, or the like), a pol II promoter (for example, a retroviral Rous sarcoma virus (RSV) LTR promoter, a cytomegalovirus (CMV) promoter, an SV40 promoter, a dihydrofolate reductase promoter, a β-acting promoter, a phosphoglycerol kinase (PGK) promoter, an EF1α promoter, or the like) or a pol I promoter.

In another specific embodiment, a CRISPR-Cas system as i) component may be present in the form of a ribonucleoprotein (RNP) in which a guide RNA and a CRISPR enzyme form a complex.

Meanwhile, in the b), the cell is sorted in which on-target effect is increased and off-target effect is decreased as identifying based on whether a selection element is expressed or not.

Moreover, a sequence of the nuclease is acquired by sequencing the nuclease obtained from the sorted cell in the c), thereby obtaining a target specific nuclease having high specificity or high activity. A nuclease used in gene editing, and the like can be prepared by using a sequence of the target specific nuclease obtained by the method.

As a method for acquiring a sequence of the obtained nuclease, for example, the following method can be performed.

That is, for example, when colonies are produced to sizes which can be seen by the unaided eye by culturing *E. coli*(*Escherichia coli)* subjected to electroporation in an LB agar plate including kanamycin, chloramphenicol, and arabinose at 30°C for 16 hours, each one of the colonies is inoculated into 10 ml of a LB buffer including chloramphenicol, and then cultured in a shaking incubator at 42°C for 12 hours. After *E*. *coli* pellets are obtained through centrifugation, a plasmid is extracted from the pellets by using a miniprep kit. The extracted plasmid can be sequenced by using, for example, a Sanger sequencing method. In the case of a primer, it is possible to use a universal sequencing primer such as CMV-F and BGH-R present outside of the Cas 9 N-term and C-term and sequencing primers in the middle part of Cas 9.

### Screening Kit

Further, the present invention provides a screening kit for selecting a target specific nuclease having high specificity or high activity, including: multiple targets containing one or more selection elements. The kit may be in the form of a composition.

In this case, the multiple targets include one or more on-targets and one or more off-targets, and a description thereof is the same as that described above.

If necessary, the kit of the present invention may be provided as a one liquid-type kit including multiple targets containing one or more selection elements.

If necessary, the kit of the present invention may be provided as a two liquid-type kit respectively including multiple targets containing one or more selection elements and a host cell.

In this case, the kit may also be provided as a one liquid-type by including multiple targets including a selection element in a host cell. For example, the kit may also be included as a separate plasmid in a host cell, and may also be provided by incorporation into a host cell genome.

If necessary, the kit of the present invention may be provided as a three liquid-type kit respectively including multiple targets containing one or more selection elements, a host cell, and a test nuclease.

In this case, the kit may also be provided as a two liquid-type by including multiple targets including a selection element in a host cell. For example, the kit may also be included as a separate plasmid in a host cell, and may also be provided by incorporation into a host cell genome.

As described above, the present invention relates to a system for selecting a target specific nuclease system having high specificity and high activity and, preferably, the present invention includes both a system for screening and selecting a target specific nuclease system by using a multiple target system capable of simultaneously identifying off-target activity and on-target activity and various uses of the same.

The characteristics and advantages of the present invention will be more understood by referencing the following detailed description which describes exemplary embodiments in which the principle of the present invention is used. These and other embodiments are described or apparent from the following detailed description and included thereby.

### [Advantageous Effects]

There still remains an off-target problem occurring at the time of correcting or manipulating a gene by using a target specific nuclease. In order to overcome the problem, it is important to develop a target specific nuclease having high specificity, which does not generate an off-target.

Thus, the present invention developed a system for screening a target specific nuclease having high specificity or high activity. The screening system of the present invention is a system capable of selecting a target specific nuclease in which an off-target effect is decreased and an on-target effect is increased, and can be usefully utilized in screening and selecting a target specific nuclease having high specificity and/or high activity, which is capable of simultaneously satisfying a decrease in the off-target effect and an increase in the on-target effect by using multiple targets.

Further, when multiple targets are designed, a selection element included in the multiple targets can be variously designed according to the purpose of the experiment, and can be utilized in selecting a target specific nuclease by using various detection methods according to the selection element.

### [Description of Drawings]

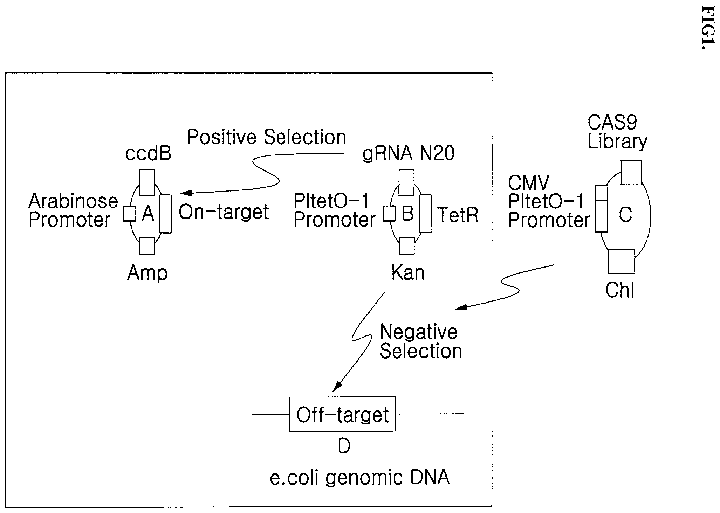

FIG. 1 schematically illustrates a screening method of a CRISPR-Cas system using multiple targets.

Plasmid A includes a target site having a toxic ccdB gene and NGG pam after an arabinose promoter. Plasmid B includes sgRNA under a PltetO-1 promoter, and can cleave a target site of Plasmid A in the presence of Cas9. An *E. coli* genomic DNA sequence is mismatched with a sgRNA sequence. Plasmid C includes a Cas9 library sequence under a CMV-pltetO-1 dual promoter.

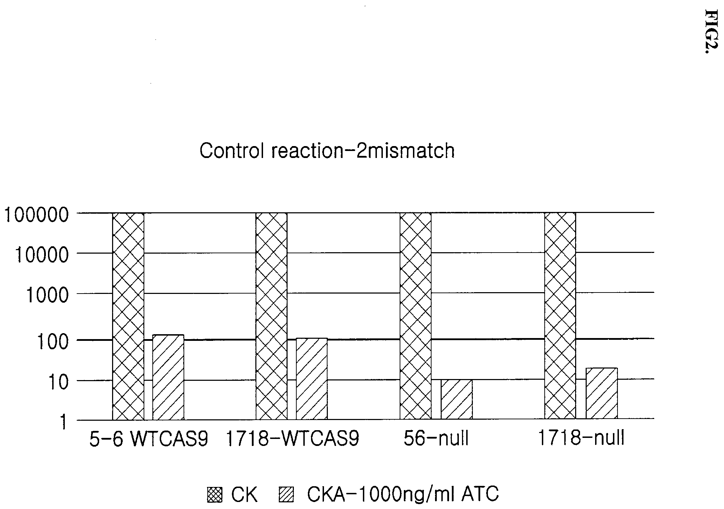

FIG. 2 is a graph illustrating screening results of a CRISPR-Cas9 system according to the design of an off-target nucleic acid sequence among multiple targets.

The graph is a result of a control experiment using an EMX-1 sequence inserted into genomic DNA of *E. coli* and Plasmid B including two mismatched sequences. The CK refers to the number of colonies formed per unit in an LB agar medium including chloramphenicol and kanamycin, and the CKA refers to the number of colonies formed per unit in an LB agar medium including chloramphenicol, kanamycin, and arabinose. When two mismatches were located near a seed region (56-WT-CAS9), less than 0.1% of the *E. coli* into which Cas9 was introduced survived. On the other hand, when a null vector, which is a negative control, was introduced into *E*. *coli* instead of Cas9, approximately 0.01% of *E*. *coli* survived. The result of the negative control is regarded as a background due to an effect of Plasmid A not acting.

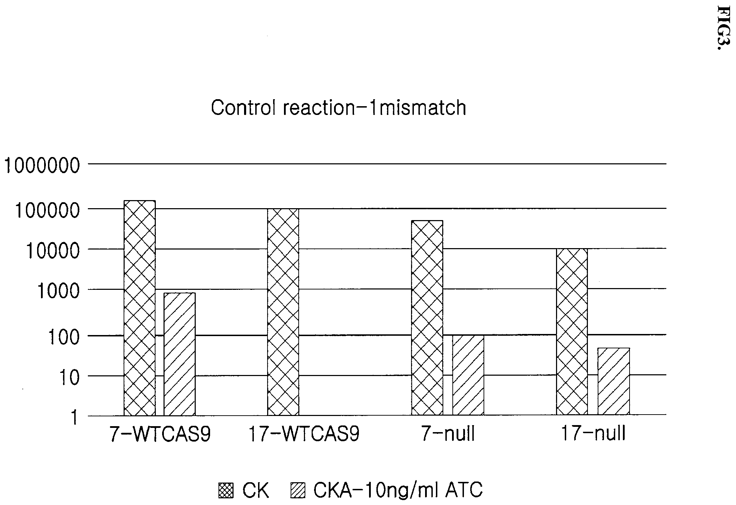

FIG. 3 is a graph illustrating screening results of a CRISPR-Cas9 system according to the design of an off-target nucleic acid sequence among multiple targets.

The graph is a result of a control experiment using an EMX-1 sequence inserted into genomic DNA of *E. coli* and Plasmid B including one mismatched sequence. In the drawing, the CK refers to the number of colonies formed per unit in an LB agar medium including chloramphenicol and kanamycin, and the CKA refers to the number of colonies formed per unit in an LB agar medium including chloramphenicol, kanamycin, and arabinose. When one mismatch was located near a seed region (7-WT-CAS9), less than 0.1% of the *E. coli* into which Cas9 was introduced survived. On the other hand, when a null vector, which is a negative control, was introduced into *E. coli* instead of Cas9, approximately 0.01% of *E. coli* survived. The result of the negative control is regarded as a background due to an effect of Plasmid A not acting.

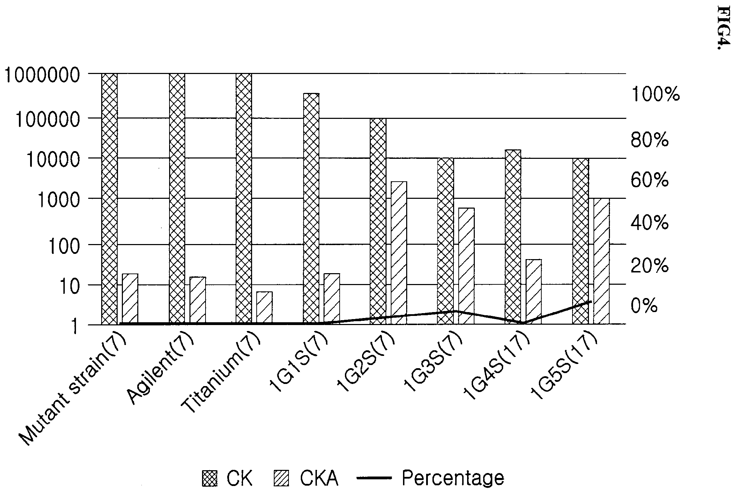

FIG. 4 is a graph illustrating screening results of Cas9 variants of a CRISPR-Cas9 system using multiple targets.

Screening of library of Plasmids A and B, BW251414-EMX1(7) including one mismatch (7), and three types (Mutant strain: derived from XL-1 Red competent cells manufactured by Agilent Technologies, Inc. Agilent: Genemorph II error prone PCR kit manufactured by Agilent Technologies, Inc. Titanium: Diversify PCR random mutagenesis kit manufactured by Clontech, Inc.). 1G1S(7) (1 Generation I Series): Library screening result for BW25141-EMX1 (7). 1G2S(7): Screening result for BW25141-EMX1 (7) of 1G1S(7) screening. 1G3S(7): Screening result for BW25141-EMX1 (7) of 1G2S(7) screening. 1G4S(17): Screening result for BW25141-EMX1 (17) of 1G3S(7) screening. 1G5S(17): Screening result for BW25141-EMX1 (17) of 1G4S(17) screening.

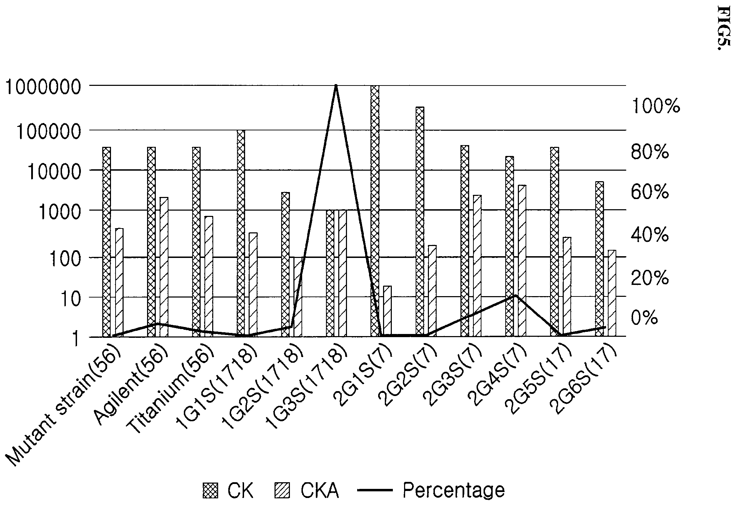

FIG. 5 is a graph illustrating screening results of Cas9 variants of a CRISPR-Cas9 system using multiple targets.

Screening of library of Plasmids A and B, BW251414-EMX1(56) including two mismatches (56), and three types (Mutant strain: derived from XL-1 Red competent cells manufactured by Agilent Technologies, Inc. Agilent: Genemorph II error prone PCR kit manufactured by Agilent Technologies, Inc. Titanium: Diversify PCR random mutagenesis kit manufactured by Clontech, Inc.). 1G1S(1718) (1Generation1Series): Library screening result for BW25141-EMX1 (1718). 1G2S(1718): Screening result for BW25141-EMX1 (1718) of 1G1S(1718) screening. 1G3S(1718): Screening result for BW25141-EMX1 (1718) of 1G2S(1718) screening. 2G1S(7): Screening result for BW25141-EMX1 (7) of shuffled library of 1G3S(17). 2G2S(7): Screening result for BW25141-EMX1 (7) of 2G1S(7) screening. 2G3S(7): Screening result for BW25141-EMX1 (7) of 2G2S(7) screening. 2G4S(7): Screening result for BW25141-EMX1 (7) of 2G3S(7) screening. 2G5S(17): Screening result for BW25141-EMX1 (17) of 2G4S(7) screening. 2G6S(17): Screening result for BW25141-EMX1 (17) of 2G5S(17) screening.

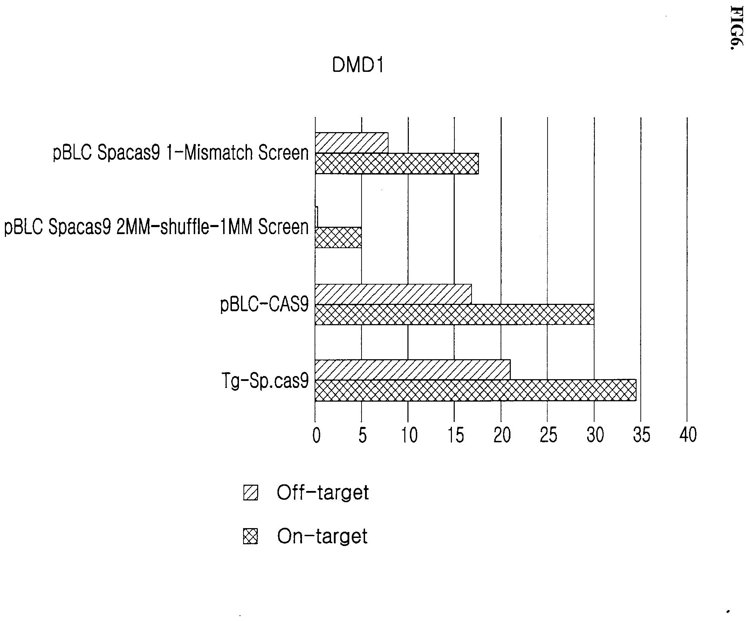

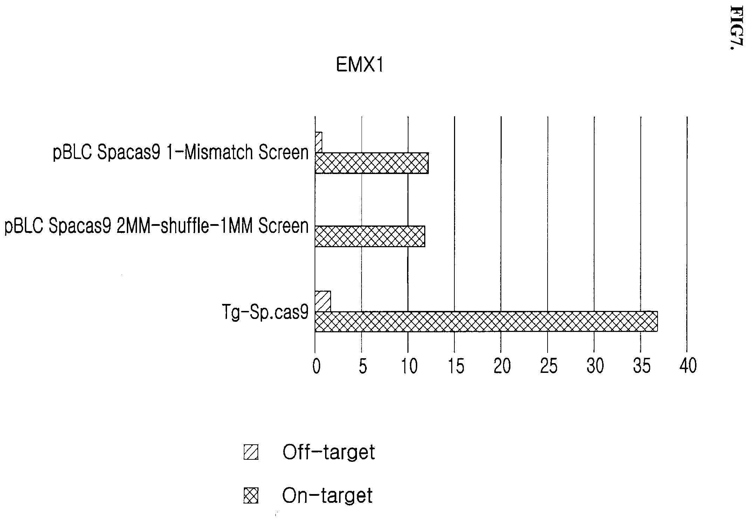

FIGS. 6 and 7 are graphs illustrating results of identifying decreased off-target activities of Cas9 variants of the CRISPR-Cas9 system via multiple targets for specific genes (EMX1 and T-DMD),
illustrating the result of measuring on-target and off-target activities of a DMD gene by using a full-state library obtained by screening performed simultaneously by two methods (FIG. 6), and
illustrating the result of measuring on-target and off-target activities of an EMX1 gene by using a full-state library obtained by screening performed simultaneously by two methods (FIG. 7).

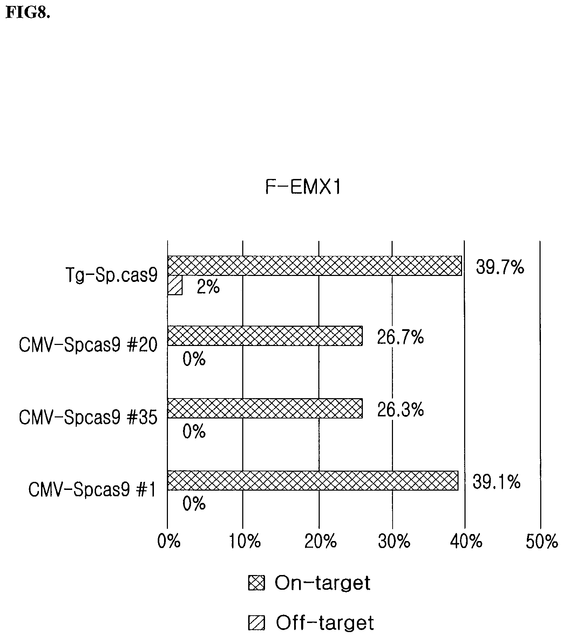

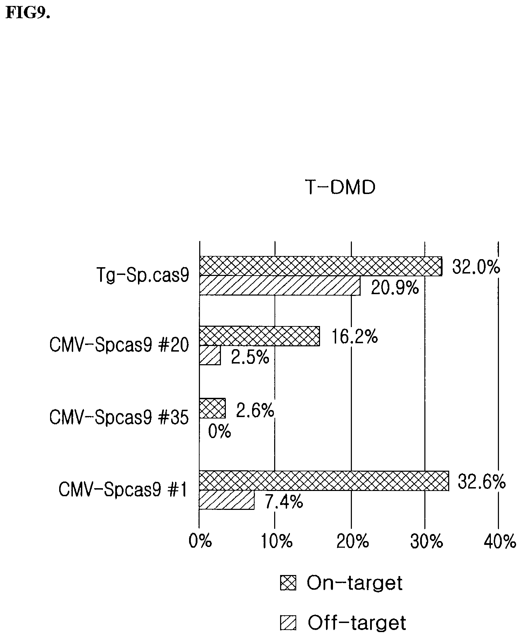

FIGS. 8 and 9 are results obtained by performing on-target and off-target experiments on each of a DMD gene and an EMX1 gene in mammalian cells by using three clones selected through screening, and results of identifying improved specificity,
illustrating on-target and off-target activities of three different clones (#1, #20 and #35) targeting human EMX-1 in HEK 293t cells (FIG. 8), and
illustrating on-target and off-target activities of three different clones (#1, #20 and #35) targeting human T-DMD in HEK 293t cells (FIG. 9).

### [Modes of the Invention]

### Definition of Terms

The term(s) "cleave", "cleavage", and/or "cleaving" refer(s) to an action which produces a cleavage in a specific nucleic acid. The cleavage (break) may leave a blunt end or sticky end (that is, a 5' or 3' overhang) as understood by a person skilled in the art. The term also includes a single-stranded DNA cleavage ("nick") and a double-stranded DNA cleavage.

The term "recombinant cell" refers to a host cell produced by genetically modifying a mother cell using genetic engineering technology (that is, recombinant technology). The recombinant cell may include the addition, deletion, and/or modification of a nucleotide sequence for a mother cell's genome.

The term "homology" refers to identity between two or more nucleic acid sequences, or two or more amino acid sequences. The sequence identity can be measured as % identity (or similarity or homology), and the higher the % is, the higher the identity between the sequences is. When homologs or orthologs of nucleic acid or amino acid sequences are aligned by using a standard method, the sequence identity is relatively high. For comparison, a method of aligning sequences is well-known in the art. Various programs and alignment algorithms are described in documents [references: Smith & Waterman, Adv. Appl. Math. 2:482, 1981; Needleman & Wunsch, J. Mol. Biol. 48:443, 1970; Pearson & Lipman, Proc. Natl. Acad. Sci. USA 85:2444, 1988; Higgins & Sharp, Gene, 73:237-44, 1988; Higgins & Sharp, CABIOS 5:151-3, 1989; Corpet et al., Nuc. Acids Res. 16:10881-90, 1988; Huang et al. Computer Appls. Biosc. 8, 155-65, 1992; and Pearson et al., Meth. Mol. Bio. 24:307-31, 1994. Altschul et al., J. Mol. Biol. 215:403-10, 1990], and the details on the sequence alignment method and homology calculation method are described. The documents [references: NCBI Basic Local Alignment Search Tool (BLAST), Altschul et al., J. Mol. Biol. 215:403-10, 1990] are available from various sources including the National Center for Biological Information (NCBI, National Library of Medicine, Building 38A, Room 8N805, Bethesda, Md. 20894) and the Internet in order to be used with blastp, blastn, blastx, tblastn and tblastx, which are sequence analysis programs. Additional information can be found at the NCBI website.

The term "hybridization" refers to a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may include two strands forming a duplex structure, three or more strands forming a multi-stranded complex, a single self-hybridizing strand, or any combination thereof.

The term "expression" refers to the process by which a polynucleotide is transcribed from a DNA template (for example, into an mRNA or another RNA transcript) and/or the process by which transcribed mRNA is translated into peptides, polypeptides, or proteins. Transcripts and encoded polypeptides may be collectively referred to as "gene products". If the polynucleotide is derived from genomic DNA, expression may include splicing of the mRNA in a eukaryotic cell.

The term "selection element" or "selection marker" refers to a gene that functions as a guide that identifies and selects an on-target effect and/or an off-target effect of a nuclease as described herein. The selection element is, but is not limited to, a toxin gene, a fluorescent marker, a luminescent marker, and a drug-selectable marker. The fluorescent marker may include, but is not limited to, a gene encoding fluorescent protein, for example, a green fluorescent protein (GFP), a cyan fluorescent protein (CFP), a yellow fluorescent protein (YFP), a red fluorescent protein (dsRFP), and the like. The luminescent marker may include, but is not limited to, a gene encoding luminescent protein such as luciferase. The drug-selectable marker suitable for use in the method and composition provided herein includes, but is not limited to, a resistance gene against antibiotics, for example, ampicillin, streptomycin, gentamicin, kanamycin, hygromycin, tetracycline, chloramphenicol, and neomycin. In some embodiments, the selection may be positive selection, that is, cells expressing a marker are isolated from a population, for example, to produce a concentrated cell population including a selectable marker. In another example, the selection may be negative selection, that is, a population is isolated from cells, for example, to produce a concentrated cell population which does not include a selectable marker. The separation can be performed by any convenient technique appropriate for a selectable marker used. For example, when a fluorescent marker is used, cells can be separated by fluorescence-activated cell sorting, whereas when a cell surface marker is inserted thereinto, cells can be separated from a heterogeneous population by an affinity separation technology, for example, magnetic separation, affinity chromatography, "panning" using an affinity reagent attached to a solid matrix, or other convenient techniques.

The implementation of the present invention uses a typical technique of immunology, biochemistry, chemistry, molecular biology, microbiology, cell biology, genomics and recombinant DNA, unless otherwise indicated.

Hereinafter, the present invention will be described in detail through exemplary embodiments in which the principle of the present invention is used.

### Screening System

### 1. Screening system

The present invention relates to a screening system for selecting a target specific nuclease using multiple targets including one or more on-targets and one or more off-targets.

The term "screening system for selecting a target specific nuclease" refers to a concept including all of a composition and a kit used to screen a target specific nuclease having high specificity and high activity, a method for using the same, and various intermediates derived during the processes. The invention described as the term "system" in the present specification may be interpreted as a composition or method so as to be suitable for the aspects of the corresponding inventions as long as the present invention is used for screening a desired target specific nuclease.

Therefore, an embodiment of the present disclosure is
a composition for screening to select a target specific nuclease having high specificity or high activity, the composition including:
i) a nuclease including a part capable of recognizing a target and a part capable of cleaving or modifying the recognized target; and
ii) multiple targets including one or more selection elements, in which the multiple targets include one or more on-targets and one or more off-targets.

### 1-1. Target specific nuclease: CRISPR-Cas System

The "target specific nuclease" may consist of a part capable of specifically recognizing a target and a part capable of cleaving or modifying the recognized target.

The part capable of specifically recognizing the target may vary according to the target gene or nucleic acid sequence.

The part capable of cleaving or modifying the recognized target can cleave or modify the recognized target. In this case, the part capable of specifically recognizing the target and the part capable of cleaving or modifying the recognized target may be each present separately or may be present functionally divided as a single structure.

The target specific nuclease is a clustered regularly interspaced short palindromic repeats (CRISPR)-CRISPR associated protein (Cas) system.

The CRISPR-Cas system consists of a guide RNA and a CRISPR enzyme.

In this case, the guide RNA may be a part capable of specifically recognizing a target, and the CRISPR enzyme may be a part capable of cleaving or modifying the recognized target.

### 1-1-1 Guide RNA

The guide RNA may include a nucleic acid sequence complementarily binding to a gene or nucleic acid sequence to be targeted.

The guide RNA may consist of a crRNA including a sequence complementary to a gene or nucleic acid sequence to be targeted and a tracrRNA binding to a CRISPR enzyme.

In this case, the crRNA includes a guide sequence which is a part capable of complementarily binding to a gene or nucleic acid sequence to be targeted. The guide sequence has a sequence complementary to a gene or nucleic acid sequence to be targeted, and may serve to recognize a gene or nucleic acid sequence to be targeted.

A size of the guide sequence may be 5 to 50, 5 to 40, 5 to 30, or 5 to 20 bps, but is not limited thereto. Preferably, the guide sequence may have a size of 5 to 20 bps.

The nucleic acid sequence of the guide sequence may include a nucleic acid sequence having 50 to 100% complementary binding with a sequence or position of a gene or nucleic acid to be targeted, but is not limited thereto.

Further, the crRNA includes a part having a sequence complementary to a portion of a tracrRNA, and accordingly, the crRNA may partially complementarily bind to the tracrRNA.

The guide RNA may be a dual guide RNA in which the crRNA and the tracrRNA are each independently present. Alternatively, the guide RNA may be a single guide RNA in which the crRNA and the tracrRNA are connected to each other. In this case, the single guide RNA may include a linker.

Further, the guide RNA may include only a crRNA according to the type of CRISPR enzyme.

The guide RNA may include a chemical modification. In this case, the chemical modification may include those in which a phosphorothioate linkage, a locked nucleic acid (LNA), 2'-O-methyl 3'phosphorothioate (MS) or 2'-O-methyl 3'thioPACE (MSP) is modified in one or two or more nucleic acids among nucleic acids included in the guide RNA.

The guide RNA may be a guide RNA in which a partial sequence of the 5' end is truncated.

A nucleic acid sequence of the guide RNA can be designed according to the target gene or nucleic acid sequence.

The guide RNA may be included in a vector, and in this case, the vector may include a promoter suitable for the expression of the guide RNA, such as a PltetO-1, arapBAD, rhampBAD or T7 promoter.

The guide RNA may be one artificially synthesized.

### 1-1-2 CRISPR enzyme

The CRISPR enzyme may be a nucleic acid having a sequence encoding the CRISPR enzyme.

A nucleic acid having the sequence encoding the CRISPR enzyme may be included in a vector. In this case, the vector may include a promoter suitable for the expression of a CRISPR enzyme, such as CMV or CAG.

The CRISPR enzyme may be a polypeptide or protein.

The CRISPR enzyme may be codon-optimized so as to be suitable for an object to be introduced.

The CRISPR enzyme may be a Type II CRISPR enzyme or a Type V CRISPR enzyme.

The Type II CRISPR enzyme may be Cas9.

The Type V CRISPR enzyme may be a Cpfl enzyme.

The Cas9 may be Streptococcus pyogenes Cas9 (SpCas9), Staphylococcus aureus Cas9 (SaCas9), Streptococcus thermophiles Cas9 (StCas9), Neisseria meningitides Cas9 (NmCas9), Campylobacter jejuni Cas9 (CjCas9) or orthologs thereof, but is not limited thereto. Preferably, the Cas9 may be SpCas9 or CjCas9.

The Cas9 may be an active Cas9 or an inactive Cas9.

The inactive Cas9 may include completely inactivated Cas9 and partially inactivated Cas9 (for example, a nickase).

With respect to the Cas9, one or two or more amino acids present in RuvC, HNH, REC and/or PI domains may be mutated.

The Cas9 may include the mutation(s) of one or two or more amino acids in an amino acid group consisting of D10, E762, H840, N854, N863 and D986 among amino acids of SpCas9 or an amino acid group of other Cas9 orthologs corresponding thereto.

The Cas9 may include the mutation(s) of one or two or more amino acids in an amino acid group consisting of R780, K810, K848, K855 and H982 among amino acids of SpCas9 or an amino acid group of other Cas9 orthologs corresponding thereto.

The Cas9 may include the mutation(s) of one or two or more amino acids in an amino acid group consisting of G1104, S1109, L1111, D1135, S1136, G1218, N1317, R1335 and T1337 among amino acids of SpCas9 or an amino acid group of other Cas9 orthologs corresponding thereto.

The Cpfl may be Francisella novicida Cpfl (FnCpf1), Acidaminococcus sp. Cpfl (AsCpfl), Lachnospiraceae bacterium Cpfl (LbCpf1) or orthologs thereof, but is not limited thereto.

The Cpfl may be an active Cpfl or an inactive Cpfl.

The inactive Cpfl may include completely inactivated Cpfl and partially inactivated Cpfl (for example, a nickase).

With respect to the Cpfl, one, or two or more amino acids present in RuvC, Nuc, WED, REC and/or PI domains may be mutated.

The Cpfl may include the mutation(s) of one or two or more amino acids in amino acid groups consisting of: D917, E1006 or D1255 among amino acids of FnCpf1; D908, E993 or D1263 among amino acids of AsCpfl; D832, E925, D947 or D1180 among amino acids of LbCpf1; or amino acid groups of other Cpfl orthologs corresponding thereto.

The CRISPR enzyme can recognize a protospacer adjacent motif (PAM) in a gene or nucleic acid sequence.

The PAM may vary according to the source of the CRISPA enzyme.

For example, the PAM may be 5'-NGG-3' when the CRISPR enzyme is SpCas9, the PAM may be 5'-NNAGAAW-3' (W = A or T) when the CRISPR enzyme is StCas9, the PAM may be 5'-NNNNGATT-3' when the CRISPR enzyme is NmCas9, the PAM may be 5'-NNNVRYAC-3' (V = G or C or A, R = A or G, and Y = C or T) when the CRISPR enzyme is CjCas9, and in this case, the N may be A, T, G or C; or A, U, G or C. Furthermore, the PAM may be 5' TTN-3' when the CRISPR enzyme is FnCpfl, the PAM may be 5'-TTTN-3' when the CRISPR enzyme is AsCpfl or LbCpfl, and in this case, the N may be A, T, G or C; or A, U, G or C.

The CRISPR enzyme may additionally include a functional domain. In this case, the CRISPR enzyme may be an active CRISPR enzyme or an inactive CRISPR enzyme.

The functional domain may be a heterologous functional domain (HFD).

The functional domain may be one selected from the group consisting of methylase activity, demethylase activity, transcription activation activity, transcription repression activity, transcription release factor activity, histone modification activity, RNA cleavage activity, DNA cleavage activity, nucleic acid binding activity and molecular switches (e.g., light inducible).

The functional domain may be one selected from the group consisting of methylases, demethylases, phosphases, thymidine kinases, cysteine deaminases, and cytidine deaminases.

In order to connect a functional domain to the CRISPR enzyme, it is possible to additionally include a linker between the CRISPR enzyme and the functional domain.

The linker may be (A)n, (G)n, GGGS, (GGS)n, (GGGGS)n, (EAAAK)n, SGGGS, GGSGGSGGS, SGSETPGTSESATPES, XTEN or (XP)n, and in this case, n may be 1, 2, 3, 4, 5, 6, 7, or more. However, the linker and n are not limited thereto.

The CRISPR enzyme may additionally include a nuclear localization sequence (NLS).

### 1-1-3 Mutation of CRISPR enzyme

The CRISPR enzyme may be an active CRISPR enzyme or an inactive CRISPR enzyme.

The inactive CRISPR enzyme may include a completely inactivated CRISPR enzyme and a partially inactivated CRISPR enzyme (for example, a nickase).

The CRISPR enzyme may be a mutated CRISPR enzyme. That is, the CRISPR enzyme may be a CRISPR enzyme which does not naturally occur.

In this case, the mutated CRISPR enzyme may be a CRISPR enzyme in which at least one or more amino acids of a naturally-occurring CRISPR enzyme are mutated, and the mutation may be the substitution, removal, addition, and the like of amino acids.

The mutated CRISPR enzyme may include the mutation(s) of one, or two or more amino acids among amino acids present in a RuvC domain of a naturally-occurring CRISPR enzyme.

The RuvC domain may be a RuvCI, RuvCII or RuvCIII domain.

The mutated CRISPR enzyme may include the mutation(s) of one, or two or more amino acids among amino acids present in an HNH domain of a naturally-occurring CRISPR enzyme.

The mutated CRISPR enzyme may include the mutation(s) of one, or two or more amino acids among amino acids present in a REC domain of a naturally-occurring CRISPR enzyme.

The modified CRISPR enzyme may include the mutation(s) of one, or two or more amino acids among amino acids present in a PI domain of a naturally-occurring CRISPR enzyme.

The mutated CRISPR enzyme may include the mutation(s) of one, or two or more amino acids among amino acids present in an Nuc domain of a naturally-occurring CRISPR enzyme.

The mutated CRISPR enzyme may include the mutation(s) of one, or two or more amino acids among amino acids present in a WED domain of a naturally-occurring CRISPR enzyme.

Further, the CRISPR enzyme may be a CRISPR enzyme recombined by consolidating a portion of each mutated CRISPR enzyme.

For example, it is possible to replace or recombine a RuvC domain, an HNH domain and a PI domain of a CRISPR enzyme present in a natural state (that is, a wild-type CRISPR enzyme) with a RuvC domain of CRISPR Enzyme Variant 1, an HNH domain of CRISPR Enzyme Variant 2, and a PI domain of CRISPR Enzyme Variant 3, respectively. Alternatively, it is possible to construct a new CRISPR variant different from CRISPR Enzyme Variants 1, 2, and 3 by combining amino acid nos. 1 to 500 of an amino acid sequence of CRISPR Enzyme Variant 1 (or a nucleic acid sequence encoding the same) with amino acid nos. 501 to 1000 of an amino acid sequence of CRISPR Enzyme Variant 2 (or a nucleic acid sequence encoding the same) and amino acid no. 1001 to end of an amino acid sequence of CRISPR Enzyme Variant 3 (or a nucleic acid sequence encoding the same).

As another example, a portion of CRISPR Enzyme Variant 1, for example, amino acid nos. 50 to 700 of an amino acid sequence thereof (or a nucleic acid sequence encoding the same) can be replaced or recombined with amino acid nos. 50 to 700 of an amino acid sequence of CRISPR Enzyme Variant 2 (or a nucleic acid sequence encoding the same).

In addition, the CRISPR enzyme may be a CRISPR enzyme produced by combining different CRISPR enzymes.

For example, a RuvC domain of Cas9 can be replaced or recombined with a RuvC domain of Cpfl.

Furthermore, the CRISPR enzyme may be a CRISPR enzyme variant in which a functional domain is additionally included in an active CRISPR enzyme or an inactive CRISPR enzyme.

The functional domain may be a heterologous functional domain (HFD)

The functional domain may be one selected from the group consisting of methylase activity, demethylase activity, transcription activation activity, transcription repression activity, transcription release factor activity, histone modification activity, RNA cleavage activity, DNA cleavage activity, nucleic acid binding activity and molecular switches (e.g., light inducible).

The functional domain may be one selected from the group consisting of methylases, demethylases, phosphases, thymidine kinases, cysteine deaminases, and cytidine deaminases.

In order to connect a functional domain to the CRISPR enzyme, it is possible to additionally include a linker between the CRISPR enzyme and the functional domain.

The linker may be (A)n, (G)n, GGGS, (GGS)n, (GGGGS)n, (EAAAK)n, SGGGS, GGSGGSGGS, SGSETPGTSESATPES, XTEN or (XP)n, and in this case, n may be 1, 2, 3, 4, 5, 6, 7, or more. However, the linker and n are not limited thereto.

For example, the CPISPR enzyme variant includes a transcription repression domain in an inactive enzyme, and the CPISPR enzyme variant may be a variant that suppresses the expression of a target gene or nucleic acid sequence. In addition, the CRISPR enzyme variant includes a cytidine deaminase in an inactive enzyme, and the CRISPR enzyme variant may be a variant capable of suppressing or increasing the expression of the corresponding gene or nucleic acid sequence by changing a specific base sequence of a target gene or nucleic acid sequence.

### 1-1-4 Mutation effect of CRISPR enzyme

The mutated CRISPR enzyme may be one mutated so as to increase a cleavage effect on a gene or nucleic acid to be targeted (that is, an on-target).

The mutated CRISPR enzyme may be one mutated so as to decrease a cleavage effect on a gene or nucleic acid to be targeted (that is, an on-target).

The mutated CRISPR enzyme may be one mutated so as to increase a cleavage effect on a gene or nucleic acid which is not targeted (that is, an off-target).

The mutated CRISPR enzyme may be one mutated so as to decrease a cleavage effect on a gene or nucleic acid which is not targeted (that is, an off-target).

The modified CRISPR enzyme may be one modified so as to increase the ability to be bound to a gene or nucleic acid.

The modified CRISPR enzyme may be one modified so as to decrease the ability to be bound to a gene or nucleic acid.

The modified CRISPR enzyme may be one modified so as to increase the ability to recognize a protospacer adjacent motif (PAM) in a gene or nucleic acid sequence.

The modified CRISPR enzyme may be one modified so as to decrease the ability to recognize a PAM in a gene or nucleic acid sequence.

The modified CRISPR enzyme may be one in which helicase kinetics is modified.

The modified CRISPR enzyme may include a conformation rearrangement according to the modification.

### 1-2. CRISPR complex

The CRISPR enzyme and the guide RNA may form a CRISPR complex.

The CRISPR complex may be formed outside a cell.

The CRISPR complex may be formed in the cytoplasm in a cell.

The CRISPR complex may be formed in the nucleus in a cell.

In the CRISPR complex, the CRISPR enzyme can recognize a PAM present in a gene or nucleic acid sequence to be targeted.

In the CRISPR complex, a guide RNA can complementarily bind to a gene or nucleic acid sequence to be targeted.

When the CRISPR complex binds to a gene or nucleic acid sequence to be targeted, the gene or nucleic acid sequence to be targeted can be cleaved or modified by the CRISPR enzyme of the CRISPR complex.

In this case, the target specific nuclease can be introduced into a cell.

The introduction of the target specific nuclease into the cell can be carried out by transfection using a virus vector system, a ribonucleoprotein (RNP), nanoparticles, a liposome, and the like, microinjection, electroporation, and the like, but the introduction method is not limited thereto.

The cell may be a prokaryotic cell or a eukaryotic cell.

### 1-3 Multiple targets

The target may be a gene or nucleic acid sequence targeted by a target specific nuclease.

The multiple targets may consist of a gene or nucleic acid sequence to be targeted (that is, an on-target) and a gene or nucleic acid sequence which is not targeted (that is, an off-target).

The multiple targets may consist of one or more on-targets and one or more off-targets. As an embodiment, the multiple targets may consist of one on-target and one or more off-targets. As another embodiment, the multiple targets may consist of one on-target and one off-target.

The multiple targets may be present in a cell or introduced from exogenous sources.

When the multiple targets are present in a cell, the multiple targets may be present in a genome of a host cell. Preferably, one or more off-targets may be present in a genome of a host cell.

When the multiple targets are introduced into a cell from exogenous sources, the multiple targets can be introduced by using a vector system.

When the multiple targets are introduced into a cell from exogenous sources, the multiple targets can be introduced by using a vector system.

When the multiple targets are introduced into a cell from exogenous sources, the multiple targets can be introduced by using a different vector for each target. That is, when the multiple targets consist of one on-target and one off-target, the vector may consist of a vector including an on-target and a vector including an off-target.

The introduction of the multiple targets into the cell can be carried out by transfection using a virus vector system, nanoparticles, a liposome, and the like, microinjection, electroporation, and the like, but the introduction method is not limited thereto.

### 1-3-1 On-target

The on-target may be a sequence or position of a target gene or nucleic acid to which a target specific nuclease complementarily binds.

The on-target may be a sequence or position of a gene or nucleic acid having a sequence complementary to a nucleic acid sequence of a part capable of specifically recognizing a target of a target specific nuclease.

When the target specific nuclease is a CRISPR-Cas system, the on-target may be a sequence or position of a gene or nucleic acid complementarily binding to a guide RNA or a guide sequence of the guide RNA.

In this case, the sequence of the gene or nucleic acid complementarily binding to the guide RNA or a guide sequence of the guide RNA may be 5 to 50 bps, and further, the length thereof may be adjusted according to the length of the nucleic acid sequence of the guide RNA or a guide sequence of the guide RNA.

When the target specific nuclease is a CRISPR-Cas system, the on-target may include a PAM sequence which a CRISPR enzyme recognizes.

When the target specific nuclease is a CRISPR-Cas system, the on-target may include a nucleic acid sequence complementarily binding to a guide RNA or a guide sequence of the guide RNA and a PAM sequence which a CRISPR enzyme recognizes.

In this case, the nucleic acid sequence complementarily binding to the guide RNA or a guide sequence of the RNA of the on-target may be positioned at the 5'-end of the PAM sequence which the CRISPR enzyme recognizes.

In this case, the nucleic acid sequence complementarily binding to the guide RNA or a guide sequence of the guide RNA of the on-target may be positioned at the 3'-end of the PAM sequence which the CRISPR enzyme recognizes.

The on-target may be (N)_{5∼50}-PAM or PAM-(N)_{5∼50}, and in this case, N may be A, T, G or C; or A, U, G or C.

In addition, the on-target may be positioned in a genome in a cell.

The cell may be a prokaryotic cell or a eukaryotic cell.

In this case, the on-target may include a selection element.

The selection element is a toxin gene, an antibiotic resistance gene, a gene encoding fluorescent protein, a tagging gene, a lacZ gene or a gene encoding luciferase.

The toxin gene may be a Hok, fst, TisB, LdrD, FlmA, Ibs, TxpA/BrnT, SymE, XCV1262, CcdB, ParE, MazF, yafO, HicA, Kid, Zeta, DarT or Sxt gene, and may be preferably CcdB, but is not limited thereto.

The toxin gene may be changed according to the source of the on-target (that is, according to the origin). That is, the toxin gene can be appropriately selected and used by the person skilled in the art according to the type of host cell.

When a host is *E. coli*(*Escherichia coli),* the toxin gene ccdB can be used.

When a host cell is a mammalian cell, the toxin gene hypoxanthine phosphoribosyl transferase (HPRT) can be used.

When the host is yeast, the toxin gene URA3 can be used.

The antibiotic resistance gene may be designed variously according to the type of antibiotic.

The antibiotic may be kanamycin, spectinomycin, streptomycin, ampicillin, carbenicillin, bleomycin, erythromycin, polymyxin B, tetracycline, zeocin, puromycin, or chloramphenicol, and is not limited thereto.

The "fluorescent protein" is a protein expressing fluorescence, and may be a green fluorescent protein (GFP), a red fluorescent protein (RFP), a yellow fluorescent protein (YFP), a cyan fluorescent protein (CFP), a blue fluorescent protein (BFP), or an orange fluorescent protein (OFP), and is not limited thereto.

The tag may be AviTag, Calmodulin-tag, polyglutamate tag, E-tag, FLAG-tag, HA-tag, His-tag, Myc-tag, NE-tag, S-tag, SBP-tag, Softag 1, Softag 3, Strep-tag, TC tag, V5 tag, VSV-tag, Xpress tag, Isopeptag, SpyTag, SnoopTag, Biotin Carboxyl Carrier Protein (BCCP), Glutathione-S-transferase(GST)-tag, HaloTag, Maltose binding protein (MBP)-tag, Nus-tag, Thioredoxin-tag, chitin binding protein (CBP), thioredoxin (TRX), or poly(NANP).

In this case, when an on-target including the selection element is cleaved or modified by a target specific nuclease, the selection element may not be expressed.

### 1-3-2 Off-target

The off-target may be a sequence or position of a non-target gene or nucleic acid to which a target specific nuclease partially complementarily binds.

The off-target may be a sequence of a nucleic acid including one or more other base sequences in the on-target.

The off-target may be a sequence or position of a gene or nucleic acid having a sequence partially complementary to a nucleic acid sequence of a part capable of specifically recognizing a target of a target specific nuclease.

When the target specific nuclease is a CRISPR-Cas system, the off-target may be a sequence or position of a gene or nucleic acid partially complementarily binding to a guide RNA or a guide sequence of the guide RNA.

In this case, the sequence of the gene or nucleic acid complementarily binding to the guide RNA or a guide sequence of the guide RNA may be 5 to 50 bps, and further, the length thereof may be adjusted according to the length of the nucleic acid sequence of the guide RNA or a guide sequence of the guide RNA.

When the target specific nuclease is a CRISPR-Cas system, the off-target may include a PAM sequence which a CRISPR enzyme recognizes.

When the target specific nuclease is a CRISPR-Cas system, the off-target may include a nucleic acid sequence complementarily binding to a guide RNA or a guide sequence of the guide RNA and a PAM sequence which a CRISPR enzyme recognizes.

In this case, the nucleic acid sequence complementarily binding to the guide RNA or a guide sequence of the guide RNA of the off-target may be positioned at the 5'-end of the PAM sequence which the CRISPR enzyme recognizes.

In this case, the nucleic acid sequence complementarily binding to the guide RNA or a guide sequence of the guide RNA of the off-target may be positioned at the 3'-end of the PAM sequence which the CRISPR enzyme recognizes.

The off-target may be (N)_{5∼50}-PAM or PAM-(N)_{5∼50}, and in this case, N may be A, T, G or C; or A, U, G or C.

The off-target may be a sequence or position of a gene or nucleic acid including one or more bonds non-complementary to a nucleic acid sequence of a part of specifically recognizing a target of a target specific nuclease.

The off-target may be a sequence or position of a gene or nucleic acid including bonds which are less than 100% complementary to a nucleic acid sequence of a part of specifically recognizing a target of a target specific nuclease. The nucleic acid sequence having a sequence homology less than 100% is a nucleic acid sequence similar to the nucleic acid sequence of the on-target, and may be a nucleic acid sequence in which one or more different base sequences are included or one or more base sequences are deleted.

When the target specific nuclease is a CRISPR-Cas system, the off-target may be a sequence or position of a gene or nucleic acid including one or more bonds non-complementary to the nucleic acid sequence of a guide RNA or a guide sequence of the guide RNA.

When the target specific nuclease is a CRISPR-Cas system, the off-target may be a sequence or position of a gene or nucleic acid including bonds which are less than 100% complementary to the nucleic acid sequence of a guide RNA or a guide sequence of the guide RNA.

When the target specific nuclease is a CRISPR-Cas system, the off-target may include one or more mismatched nucleic acid sequences among PAM sequences which a CRISPR enzyme recognizes.

In this case, the off-target may be positioned in a genome in a cell.

The cell may be a prokaryotic cell or a eukaryotic cell.

In this case, the off-target may include a selection element.

The selection element is a toxin gene, an antibiotic resistance gene, a gene encoding fluorescent protein, a tagging gene, a lacZ gene or a gene encoding luciferase, and is not limited thereto.

In this case, when an off-target including the selection element is cleaved or modified by a target specific nuclease, the selection element may not be expressed.

In this case, when an off-target including the selection element is not cleaved or modified by a target specific nuclease, the selection element may be expressed.

### 1-4 Effect of selection element

The term "selection element" of the present invention refers to a marker for detecting whether a target gene or nucleic acid sequence is cleaved or modified by a target specific nuclease. Therefore, the "selection element" can be used interchangeably with "a selection marker".

Based on whether the selection element is expressed or not, it is possible to detect whether a target gene or nucleic acid sequence is cleaved or modified by a target specific nuclease. That is, it is possible to detect whether the on-target effect is increased and/or whether the off-target effect is decreased.

A selection element included in an on-target and/or an off-target may be an element which determines high specificity or high activity of a target specific nuclease.

As an example, when a target specific nuclease is screened by using an on-target including a toxin gene as a selection element and an off-target including an antibiotic resistance gene as a selection element, if respective vectors including the on-target and the off-target and a vector including the target specific nuclease are introduced into a cell, it is possible to expect that the target specific nuclease exhibiting high specificity cleaves the on-target, and as a result, the expression of the toxin gene is suppressed, whereas the off-target is not cleaved, and as a result, the antibiotic resistance gene is expressed. Therefore, a cell where only the on-target is cleaved and the off-target is not cleaved, into which a target specific nuclease having high specificity is introduced, can survive when treated with an antibiotic. Accordingly, it is possible to screen a target specific nuclease having high specificity or high activity based on whether these selection elements are expressed or not.

As another example, when a target specific nuclease is screened by using an on-target including a toxin gene as a selection element and an off-target present in a genome of a cell, if a vector including the on-target and a vector including the target specific nuclease are introduced into a cell, it is possible to expect that the target specific nuclease exhibiting high specificity cleaves the on-target, and as a result, the expression of the toxin gene is suppressed, whereas the off-target is not cleaved, and as a result, the genome is not cleaved. Therefore, a cell where only the on-target is cleaved and the off-target is not cleaved, into which a target specific nuclease having high specificity is introduced, can survive, and a cell in which both the on-target and the off-target are cleaved cannot survive because the genome is cleaved due to the cleavage of the off-target, so that the two types of cells can be distinguished from each other.

As still another example, when a target specific nuclease is screened by using an on-target including a toxin gene as a selection element and an off-target including a gene encoding fluorescent protein as a selection element, if a vector including the on-target and a vector including the target specific nuclease are introduced into a cell, it is possible to expect that the target specific nuclease exhibiting high specificity cleaves the on-target, and as a result, the expression of the toxin gene is suppressed, whereas the off-target is not cleaved, and as a result, the fluorescent protein is expressed. Therefore, a cell where only the on-target is cleaved and the off-target is not cleaved, into which a target specific nuclease having high specificity is introduced, can survive, and can also be distinguished with a fluorescent protein.

As described above, it is possible to variously design a method for screening a target specific nuclease according to the selection element.

### 2. Screening Method

A target specific nuclease having high specificity or high activity can be screened by using the screening system.

As an aspect, the present invention relates to a method for screening a target specific nuclease having high specificity or high activity among a plurality of target specific nuclease test groups.

In the present invention, the term "method for screening a target specific nuclease" refers to the sorting of a cell including a target specific nuclease having high specificity or high activity. That is, the method refers to a step prior to a step of identifying a specific nuclease.

In particular, the method is very useful in selecting a target specific nuclease having high specificity or high activity among variants of various nucleases prepared by improving a target specific nuclease and a nuclease library.

### 2-1. First embodiment of screening method

As a specific embodiment,
the method can be utilized as a method for screening a target specific nuclease having high specificity and high activity among various "variants" of a target specific nuclease.

The method is a method using:
i) multiple targets including one or more on-targets and one or more off-targets; and
ii) a target specific nuclease variant, the method including:
   a) introducing i) and ii) components into a cell; and
   b) sorting a cell in which only the on-target is modified in the a).

### 2-1-1 Components

In this case, i) component can use a vector system, and in i) component, the on-target and the off-target can be included in respective vectors.

A target specific nuclease of ii) component is a CRISPR-Cas system.

The CRISPR-Cas system consists of a guide RNA and a CRISPR enzyme.

A target specific nuclease variant of ii) component may be a variant of the CRISPR-Cas system, may be preferably a variant of the CRISPR enzyme, but is not limited thereto. The nuclease variant can be produced by electromagnetic waves, UV, radiation, chemicals, external/internal gene action, and the like. In an example, variants were obtained by irradiating a WT CRISPR-Cas system with UV.

The CRISPR enzyme may be Cas9, and a variant of the CRISPR enzyme may be a variant of Cas9.

The variant of Cas9 may be a variant in which an on-target effect is increased or decreased. In this case, the on-target effect means that an on-target position is cleaved or modified by Cas9.

The variant of Cas9 may be a variant in which an off-target effect is increased or decreased. In this case, the off-target effect means that an off-target position is cleaved or modified by Cas9.

Preferably, the variant of Cas9 may be a variant in which the on-target effect is increased and/or the off-target effect is decreased.
ii) Component can use a vector system, and when ii) component is a CRISPR-Cas system, the guide RNA and the CRISPR enzyme may be included in the same vector or different vectors.
ii) Component can use a ribonucleoprotein (RNP) system, and when ii) component is a CRISPR-Cas system, the guide RNA and the CRISPR enzyme may be in the form of a RNA-protein complex.
ii) Component may be one artificially synthesized.

When ii) component is a CRISPR-Cas system, the guide RNA may be one artificially synthesized, and the CRISPR enzyme may also be a protein or polypeptide artificially synthesized.

In i) component, the on-target may be a sequence or position of a gene or nucleic acid complementarily binding to a guide RNA or a guide sequence of the guide RNA of ii) component.

In i) component, the off-target may be a sequence or position of a gene or nucleic acid partially complementarily binding to a guide RNA or a guide sequence of the guide RNA of ii) component.

In i) component, the off-target may be a sequence or position of a gene or nucleic acid forming one or more bonds non-complementary to the nucleic acid sequence of a guide RNA or a guide sequence of the guide RNA of ii) component.

In i) component, the on-target and/or the off-target include a selection element.

The selection element is a toxin gene, an antibiotic resistance gene, a gene encoding fluorescent protein, a tagging gene, a lacZ gene or a gene encoding luciferase.

### 2-1-2 Method

In this case, when i) and ii) components are introduced into a cell in the a), i) and ii) components can be introduced into the cell by transfection using a virus vector system, a ribonucleoprotein (RNP), nanoparticles, a liposome, and the like, microinjection, electroporation, and the like, but the introduction method is not limited thereto.

The cell may be a prokaryotic cell or a eukaryotic cell.

Meanwhile, the off-target may be present in a genome of a cell.

When the off-target among i) component is present in the cell genome, a vector including the on-target of i) component and ii) component can be introduced into the cell.

Meanwhile, the on-target may be present in a genome of a cell.

When the on-target among i) component is present in the cell genome, a vector including the off-target of i) component and ii) component can be introduced into the cell.

In the b),
among cells into which i) and ii) components are introduced in the a), a cell in which only the on-target among i) component is cleaved by ii) component can be sorted.

The cell in which only the on-target is cleaved can be selected by the suppression of expression of a selection element included in the on-target.

For example, when the on-target includes a toxin gene, the cell can survive due to the suppression of expression of the toxin gene as the on-target is cleaved.

Further, the off-target among i) component can sort a cell which is not cleaved by ii) component.

The cell in which the off-target is not cleaved can be selected by the expression of the selection element because the off-target is not cleaved.

For example, when the off-target includes an antibiotic resistance gene, a cell having antibiotic resistance can be sorted in the presence of an antibiotic through the expression of the antibiotic resistance gene as the off-target is not cleaved.

Alternatively, when the off-target is positioned in a genome in a cell, the genome of the cell is not cleaved by ii) component, so that the genome of the cell is normally maintained, and as a result, the cell can survive.

In this case, the sorted cell in the b) may be a cell in which the on-target of i) component is cleaved and the off-target of i) component is not cleaved by ii) component. These cells can be distinguished by selection elements.

Accordingly, it is possible to screen a target specific nuclease having high specificity and high activity, which effectively cleaves only an on-target among various target specific nuclease variants by using the screening method as described above, and in this case, a screening method can be designed by various detection methods using various selection elements.

### 2-2. Second embodiment of screening method

As another specific embodiment,

the method can be utilized as a method for screening a target specific nuclease having high specificity and high activity in a target specific nuclease "library".

The method is a screening method using:
i) multiple targets including one or more on-targets and one or more off-targets; and
ii) a target specific nuclease library, the method including:
   a) introducing i) and ii) components into a cell; and
   b) sorting a cell in which only the on-target is modified in the a).

### 2-2-1 Components

In this case, i) component can use a vector system, and in i) component, the on-target and the off-target can be included in respective vectors.

A target specific nuclease of ii) component is a CRISPR-Cas system.

The target specific nuclease library may be a CRISPR-Cas system library. The nuclease library may be directly manufactured, may also be commercially available, and may also be obtained from a database.

The CRISPR-Cas system consists of a guide RNA and a CRISPR enzyme.

The CRISPR-Cas system library may be a guide RNA library and/or a CRISPR enzyme library.
ii) Component can use a vector system, and when ii) component is a CRISPR-Cas system, the guide RNA and the CRISPR enzyme may be included in the same vector or different vectors.
ii) Component can use a ribonucleoprotein (RNP) system, and when ii) component is a CRISPR-Cas system, the guide RNA and the CRISPR enzyme may be in the form of a RNA-protein complex.
ii) Component may be one artificially synthesized.

When ii) component is a CRISPR-Cas system, the guide RNA may be one artificially synthesized, and the CRISPR enzyme may also be a protein or polypeptide artificially synthesized.

In i) component, the on-target may be a sequence or position of a gene or nucleic acid complementarily binding to a guide RNA or a guide sequence of the guide RNA of ii) component.

In i) component, the off-target may be a sequence or position of a gene or nucleic acid partially complementarily binding to a guide RNA or a guide sequence of the guide RNA of ii) component.

In component i), the off-target may be a sequence or position of a gene or nucleic acid forming one or more bonds non-complementary to the nucleic acid sequence of a guide RNA or a guide sequence of the guide RNA of ii) component.

In i) component, the on-target and/or the off-target include a selection element.

The selection element is a toxin gene, an antibiotic resistance gene, a gene encoding fluorescent protein, a tagging gene, a lacZ gene or a gene encoding luciferase.

### 2-2-2 Method

In this case, when i) and ii) component are introduced into the cell in the a), i) and ii) components can be introduced into the cell by transfection using a virus vector system, a ribonucleoprotein (RNP), nanoparticles, a liposome, and the like, microinjection, electroporation, and the like, but the introduction method is not limited thereto.

The cell may be a prokaryotic cell or a eukaryotic cell.

Meanwhile, the off-target may be present in a genome of a cell.

When the off-target among i) component is present in the cell genome, a vector including the on-target of i) component and ii) component can be introduced into the cell.

Meanwhile, the on-target may be present in a genome of a cell.

When the on-target among i) component is present in the cell genome, a vector including the off-target of i) component and ii) component can be introduced into the cell.

In the b),
among cells into which i) and ii) components are introduced in the a), a cell in which only the on-target among i) component is cleaved by ii) component can be sorted.

The cell in which only the on-target is cleaved can be selected by the suppression of expression of a selection element included in the on-target.

For example, when the on-target includes a toxin gene, the cell can survive due to the suppression of expression of the toxin gene as the on-target is cleaved.

Further, the off-target among i) component can sort a cell which is not cleaved by ii) component.

The cell in which the off-target is not cleaved can be selected by the expression of the selection element because the off-target is not cleaved.

For example, when the off-target includes an antibiotic resistance gene, a cell having antibiotic resistance can be sorted in the presence of an antibiotic through the expression of the antibiotic resistance gene as the off-target is not cleaved.

Alternatively, when the off-target is positioned in a genome in a cell, the genome of the cell is not cleaved by ii) component, so that the genome of the cell is normally maintained, and as a result, the cell can survive.

In this case, the sorted cell in the b) may be a cell in which the on-target of i) component is cleaved and the off-target of i) component is not cleaved by ii) component. These cells can be distinguished by selection elements.

Accordingly, it is possible to screen a target specific nuclease having high specificity or high activity in a target specific nuclease library through the selected cell by using the screening method as described above, and in this case, a screening method can be designed by various detection methods using various selection elements.

### Selection System

### 3. Selection system of target specific nuclease

A cell including a target specific nuclease having high specificity or high activity is sorted from test nucleases by using the screening system, and furthermore, a desired nuclease can be selected (identified) therefrom.

Another aspect of the present invention relates to a selection system of a target specific nuclease using multiple targets including one or more on-targets and one or more off-targets.

The term "selection system of a target specific nuclease" refers to a concept including all of a composition and a kit used to select a target specific nuclease having high specificity and high activity and identify a specific constitution thereof, a method for using the same, and various intermediates derived during the processes. The invention described as a "system" in the present specification may be interpreted as a composition or method so as to be suitable for the aspects of the corresponding inventions as long as the present invention is used for selecting a desired target specific nuclease.

The selection system may consist of multiple targets and a target specific nuclease.

### 3-1. Target specific nuclease (CRISPR-Cas system)

In this case, the target specific nuclease may consist of a part capable of specifically recognizing a target and a part capable of cleaving or modifying the recognized target.

The part capable of specifically recognizing the target may vary according to the target gene or nucleic acid sequence.

The part capable of cleaving or modifying the recognized target can cleave or modify the recognized target. In this case, the part capable of specifically recognizing the target and the part capable of cleaving or modifying the recognized target may be each present separately or may be present functionally divided as a single structure.

The target specific nuclease is a clustered regularly interspaced short palindromic repeats (CRISPR)-CRISPR associated protein (Cas) system.

The CRISPR-Cas system consists of a guide RNA and a CRISPR enzyme.

In this case, the guide RNA may be a part capable of specifically recognizing a target, and the CRISPR enzyme may be a part capable of cleaving or modifying the recognized target.

### 3-1-1 Guide RNA

The guide RNA may include a nucleic acid sequence complementarily binding to a gene or nucleic acid sequence to be targeted.

The guide RNA may consist of a crRNA including a sequence complementary to a gene or nucleic acid sequence to be targeted and a tracrRNA binding to a CRISPR enzyme.

In this case, the crRNA includes a guide sequence which is a part capable of complementarily binding to a gene or nucleic acid sequence to be targeted. The guide sequence has a sequence complementary to a gene or nucleic acid sequence to be targeted, and may serve to recognize a gene or nucleic acid sequence to be targeted.

A size of the guide sequence may be 5 to 50, 5 to 40, 5 to 30, or 5 to 20 bps, but is not limited thereto. Preferably, the guide sequence may have a size of 5 to 20 bps.

The nucleic acid sequence of the guide sequence may include a nucleic acid sequence having 50 to 100% complementary binding with a sequence or position of a gene or nucleic acid to be targeted, but is not limited thereto.

Further, the crRNA includes a part having a sequence complementary to a portion of a tracrRNA, and accordingly, the crRNA may partially complementarily bind to the tracrRNA.

The guide RNA may be a dual guide RNA in which the crRNA and the tracrRNA are each separately present. Alternatively, the guide RNA may be a single guide RNA in which the crRNA and the tracrRNA are connected to each other. In this case, the single guide RNA may include a linker.

Further, the guide RNA may include only a crRNA according to the type of CRISPR enzyme.

The guide RNA may include a chemical modification. In this case, the chemical modification may include those in which a phosphorothioate linkage, a locked nucleic acid (LNA), 2'-O-methyl 3'phosphorothioate (MS) or 2'-O-methyl 3'thioPACE (MSP) is modified in one or two or more nucleic acids among nucleic acids included in the guide RNA.

The guide RNA may be a guide RNA in which a partial sequence of the 5' end is truncated.

A nucleic acid sequence of the guide RNA can be designed according to the target gene or nucleic acid sequence.

The guide RNA may be included in a vector, and in this case, the vector may include a promoter suitable for the expression of the guide RNA, such as a PltetO-1, arapBAD, rhampBAD or T7 promoter.

The guide RNA may be one artificially synthesized.

### 3-1-2 CRISPR enzyme

The CRISPR enzyme may be a nucleic acid having a sequence encoding the CRISPR enzyme.

A nucleic acid having the sequence encoding the CRISPR enzyme may be included in a vector. In this case, the vector may include a promoter suitable for the expression of a CRISPR enzyme, such as CMV or CAG.

The CRISPR enzyme may be a polypeptide or protein.

The CRISPR enzyme may be codon-optimized so as to be suitable for an object to be introduced.

The CRISPR enzyme may be a Type II CRISPR enzyme or a Type V CRISPR enzyme.

The Type II CRISPR enzyme may be Cas9.

The Type V CRISPR enzyme may be a Cpfl enzyme.

The Cas9 may be Streptococcus pyogenes Cas9 (SpCas9), Staphylococcus aureus Cas9 (SaCas9), Streptococcus thermophiles Cas9 (StCas9), Neisseria meningitides Cas9 (NmCas9), Campylobacter jejuni Cas9 (CjCas9) or orthologs thereof, but is not limited thereto. Preferably, the Cas9 may be SpCas9 or CjCas9

The Cas9 may be an active Cas9 or an inactive Cas9.

The inactive Cas9 may include a completely inactivated Cas9 and a partially inactivated Cas9 (for example, a nickase).

With respect to the Cas9, one, or two or more amino acids present in RuvC, HNH, REC and/or PI domains may be mutated.

The Cas9 may include the mutation(s) of one or two or more amino acids in an amino acid group consisting of D10, E762, H840, N854, N863 and D986 among amino acids of SpCas9 or an amino acid group of other Cas9 ortholog corresponding thereto.

The Cas9 may include the mutation(s) of one or two or more amino acids in an amino acid group consisting of R780, K810, K848, K855 and H982 among amino acids of SpCas9 or an amino acid group of other Cas9 ortholog corresponding thereto.

The Cas9 may include the mutation(s) of one or two or more amino acids in an amino acid group consisting of G1104, S1109, L1111, D1135, S1136, G1218, N1317, R1335 and T1337 among amino acids of SpCas9 or an amino acid group of other Cas9 ortholog corresponding thereto.

The Cpfl may be Francisella novicida Cpfl (FnCpf1), Acidaminococcus sp. Cpfl (AsCpfl), Lachnospiraceae bacterium Cpfl (LbCpf1) or orthologs thereof, but is not limited thereto.

The Cpfl may be an active Cpfl or an inactive Cpfl.

The inactive Cpfl may include a completely inactivated Cpfl and a partially inactivated Cpfl (for example, a nickase).

With respect to the Cpfl, one, or two or more amino acids present in RuvC, Nuc, WED, REC and/or PI domains may be mutated.

The Cpfl may include the mutation(s) of one or two or more amino acids in amino acid groups consisting of: D917, E1006 or D1255 among amino acids of FnCpf1; D908, E993 or D1263 among amino acids of AsCpfl; D832, E925, D947 or D1180 among amino acids of LbCpf1; or amino acid groups of other Cpfl orthologs corresponding thereto.

The CRISPR enzyme can recognize a protospacer adjacent motif (PAM) in a gene or nucleic acid sequence.

The PAM may vary according to the source of the CRISPA enzyme.

For example, the PAM may be 5'-NGG-3' when the CRISPR enzyme is SpCas9, the PAM may be 5'-NNAGAAW-3' (W = A or T) when the CRISPR enzyme is StCas9, the PAM may be 5'-NNNNGATT-3' when the CRISPR enzyme is NmCas9, the PAM may be 5'-NNNVRYAC-3' (V = G or C or A, R = A or G, and Y = C or T) when the CRISPR enzyme is CjCas9, and in this case, the N may be A, T, G or C; or A, U, G or C. Furthermore, the PAM may be 5' TTN-3' when the CRISPR enzyme is FnCpfl, the PAM may be 5'-TTTN-3' when the CRISPR enzyme is AsCpfl or LbCpfl, and in this case, the N may be A, T, G or C; or A, U, G or C.

The CRISPR enzyme may additionally include a functional domain. In this case, the CRISPR enzyme may be an active CRISPR enzyme or an inactive CRISPR enzyme.

The functional domain may be a heterologous functional domain (HFD)

The functional domain may be one selected from the group consisting of methylase activity, demethylase activity, transcription activation activity, transcription repression activity, transcription release factor activity, histone modification activity, RNA cleavage activity, DNA cleavage activity, nucleic acid binding activity and molecular switches (e.g., light inducible).

The functional domain may be one selected from the group consisting of methylases, demethylases, phosphases, thymidine kinases, cysteine deaminases, and cytidine deaminases.

In order to connect a functional domain to the CRISPR enzyme, it is possible to additionally include a linker between the CRISPR enzyme and the functional domain.

The linker may be (A)n, (G)n, GGGS, (GGS)n, (GGGGS)n, (EAAAK)n, SGGGS, GGSGGSGGS, SGSETPGTSESATPES, XTEN or (XP)n, and in this case, n may be 1, 2, 3, 4, 5, 6, 7, or more. However, the linker and n are not limited thereto.

The CRISPR enzyme may additionally include a nuclear localization sequence (NLS).

### 3-1-3 Modification of CRISPR enzyme

The CRISPR enzyme may be an active CRISPR enzyme or an inactive CRISPR enzyme.

The inactive CRISPR enzyme may include a completely inactivated CRISPR enzyme and a partially inactivated CRISPR enzyme (for example, a nickase).

The CRISPR enzyme may be a mutated CRISPR enzyme. That is, the CRISPR enzyme may be a CRISPR enzyme which does not naturally occur.

In this case, the mutated CRISPR enzyme may be a CRISPR enzyme in which at least one or more amino acids of a naturally-occurring CRISPR enzyme are mutated, and the mutation may be the substitution, removal, addition, and the like of amino acids.

The mutated CRISPR enzyme may include the mutation(s) of one, or two or more amino acids among amino acids present in a RuvC domain of a naturally-occurring CRISPR enzyme.

The RuvC domain may be a RuvCI, RuvCII or RuvCIII domain.

The mutated CRISPR enzyme may include the mutation(s) of one, or two or more amino acids among amino acids present in an HNH domain of a naturally-occurring CRISPR enzyme.

The mutated CRISPR enzyme may include the mutation(s) of one, or two or more amino acids among amino acids present in a REC domain of a naturally-occurring CRISPR enzyme.

The mutated CRISPR enzyme may include the mutation(s) of one, or two or more amino acids among amino acids present in a PI domain of a naturally-occurring CRISPR enzyme.

The mutated CRISPR enzyme may include the mutation(s) of one, or two or more amino acids among amino acids present in a Nuc domain of a naturally-occurring CRISPR enzyme.

The mutated CRISPR enzyme may include the mutation(s) of one, or two or more amino acids among amino acids present in a PI domain of a naturally-occurring CRISPR enzyme.

Further, the CRISPR enzyme may be a CRISPR enzyme recombined by consolidating a portion of each mutated CRISPR enzyme.

For example, it is possible to replace or recombine a RuvC domain, an HNH domain and a PI domain of a CRISPR enzyme present in a natural state (that is, a wild-type CRISPR enzyme) with a RuvC domain of CRISPR Enzyme Variant 1, an HNH domain of CRISPR Enzyme Variant 2, and a PI domain of CRISPR Enzyme Variant 3, respectively. Alternatively, it is possible to construct a new CRISPR variant different from CRISPR Enzyme Variants 1, 2, and 3 by combining amino acid nos. 1 to 500 of an amino acid sequence of CRISPR Enzyme Variant 1 (or a nucleic acid sequence encoding the same) with amino acid nos. 501 to 1000 of an amino acid sequence of CRISPR Enzyme Variant 2 (or a nucleic acid sequence encoding the same) and amino acid no. 1001 to end of an amino acid sequence of CRISPR Enzyme Variant 3 (or a nucleic acid sequence encoding the same).

As another example, a portion of CRISPR Enzyme Variant 1, for example, amino acid nos. 50 to 700 of an amino acid sequence thereof (or a nucleic acid sequence encoding the same) can be replaced or recombined with amino acid nos. 50 to 700 of an amino acid sequence of CRISPR Enzyme Variant 2 (or a nucleic acid sequence encoding the same).

In addition, the CRISPR enzyme may be a CRISPR enzyme produced by combining different CRISPR enzymes.

For example, a RuvC domain of Cas9 can be replaced or recombined with a RuvC domain of Cpfl.

Furthermore, the CRISPR enzyme may be a CRISPR enzyme variant in which a functional domain is additionally included in an active CRISPR enzyme or an inactive CRISPR enzyme.

The functional domain may be a heterologous functional domain (HFD)

The functional domain may be one selected from the group consisting of methylase activity, demethylase activity, transcription activation activity, transcription repression activity, transcription release factor activity, histone modification activity, RNA cleavage activity, DNA cleavage activity, nucleic acid binding activity and molecular switches (e.g., light inducible).

The functional domain may be one selected from the group consisting of methylases, demethylases, phosphases, thymidine kinases, cysteine deaminases, and cytidine deaminases.

In order to connect a functional domain to the CRISPR enzyme, it is possible to additionally include a linker between the CRISPR enzyme and the functional domain.

The linker may be (A)n, (G)n, GGGS, (GGS)n, (GGGGS)n, (EAAAK)n, SGGGS, GGSGGSGGS, SGSETPGTSESATPES, XTEN or (XP)n, and in this case, n may be 1, 2, 3, 4, 5, 6, 7, or more. However, the linker and n are not limited thereto.

For example, the CPISPR enzyme variant includes a transcription repression domain in an inactive enzyme, and the CPISPR enzyme variant may be a variant that suppresses the expression of a target gene or nucleic acid sequence. In addition, the CRISPR enzyme variant includes a cytidine deaminase in an inactive enzyme, and the CRISPR enzyme variant may be a variant capable of suppressing or increasing the expression of the corresponding gene or nucleic acid sequence by changing a specific base sequence of a target gene or nucleic acid sequence.

### 3-1-4 Mutation effect of CRISPR enzyme

The mutated CRISPR enzyme may be one mutated so as to increase a cleavage effect on a gene or nucleic acid to be targeted (that is, an on-target).

The mutated CRISPR enzyme may be one mutated so as to decrease a cleavage effect on a gene or nucleic acid to be targeted (that is, an on-target).

The mutated CRISPR enzyme may be one mutated so as to increase a cleavage effect on a gene or nucleic acid which is not targeted (that is, an off-target).

The mutated CRISPR enzyme may be one mutated so as to decrease a cleavage effect on a gene or nucleic acid which is not targeted (that is, an off-target).

The modified CRISPR enzyme may be one modified so as to increase the ability to be bound to a gene or nucleic acid.

The modified CRISPR enzyme may be one modified so as to decrease the ability to be bound to a gene or nucleic acid.

The modified CRISPR enzyme may be one modified so as to increase the ability to recognize a protospacer adjacent motif (PAM) in a gene or nucleic acid sequence.

The modified CRISPR enzyme may be one modified so as to decrease the ability to recognize a PAM in a gene or nucleic acid sequence.

The modified CRISPR enzyme may be one in which the helicase kinetics is modified.

The modified CRISPR enzyme may include a conformation rearrangement according to the modification.

### 3-2. CRISPR complex

The CRISPR enzyme and the guide RNA may form a CRISPR complex.

The CRISPR complex may be formed outside a cell.

The CRISPR complex may be formed in the cytoplasm in a cell.

The CRISPR complex may be formed in the nucleus in a cell.

In the CRISPR complex, the CRISPR enzyme can recognize a PAM present in a gene or nucleic acid sequence to be targeted.

In the CRISPR complex, a guide RNA can complementarily bind to a gene or nucleic acid sequence to be targeted.

When the CRISPR complex binds to a gene or nucleic acid sequence to be targeted, the gene or nucleic acid sequence to be targeted can be cleaved or modified by the CRISPR enzyme of the CRISPR complex.

In this case, the target specific nuclease can be introduced into a cell.

The introduction of the target specific nuclease into the cell can be carried out by transfection using a virus vector system, a ribonucleoprotein (RNP), nanoparticles, a liposome, and the like, microinjection, electroporation, and the like, but the introduction method is not limited thereto.

The cell may be a prokaryotic cell or a eukaryotic cell.

### 3-3 Multiple targets

In this case, the target may be a gene or nucleic acid sequence targeted by a target specific nuclease.

The multiple targets may consist of a gene or nucleic acid sequence to be targeted (that is, an on-target) and a gene or nucleic acid sequence which is not targeted (that is, an off-target).

The multiple targets may consist of one or more on-targets and one or more off-targets. As an embodiment, the multiple targets may consist of one on-target and one or more off-targets. As another embodiment, the multiple targets may consist of one on-target and one off-target.

The multiple targets may be present in a cell or introduced from exogenous sources.

When the multiple targets are present in a cell, the multiple targets may be present in a genome of a host cell. Preferably, one or more off-targets may be present in a genome of a host cell.

When the multiple targets are introduced into a cell from exogenous sources, the multiple targets can be introduced by using a vector system.

When the multiple targets are introduced into a cell from exogenous sources, the multiple targets can be introduced by using a different vector for each target. That is, when the multiple targets consist of one on-target and one off-target, the vector may consist of a vector including an on-target and a vector including an off-target.

The introduction of the multiple targets into the cell can be carried out by transfection using a virus vector system, nanoparticles, a liposome, and the like, microinjection, electroporation, and the like, but the introduction method is not limited thereto.

### 3-3-1 On-target

The on-target may be a sequence or position of a target gene or nucleic acid to which a target specific nuclease complementarily binds.

The on-target may be a sequence or position of a gene or nucleic acid having a sequence complementary to a nucleic acid sequence of a part capable of specifically recognizing a target of a target specific nuclease.

When the target specific nuclease is a CRISPR-Cas system, the on-target may be a sequence or position of a gene or nucleic acid complementarily binding to a guide RNA or a guide sequence of the guide RNA.

In this case, the sequence of the gene or nucleic acid complementarily binding to the guide RNA or a guide sequence of the guide RNA may be 5 to 50 bps, and further, the length thereof may be adjusted according to the length of the nucleic acid sequence of the guide RNA or a guide sequence of the guide RNA.

When the target specific nuclease is a CRISPR-Cas system, the on-target may include a PAM sequence which a CRISPR enzyme recognizes.

When the target specific nuclease is a CRISPR-Cas system, the on-target may include a nucleic acid sequence complementarily binding to a guide RNA or a guide sequence of the guide RNA and a PAM sequence which a CRISPR enzyme recognizes.

In this case, the nucleic acid sequence complementarily binding to the guide RNA or a guide sequence of the guide RNA of the on-target may be positioned at the 5'-end of the PAM sequence which the CRISPR enzyme recognizes.

In this case, the nucleic acid sequence complementarily binding to the guide RNA or a guide sequence of the guide RNA of the on-target may be positioned at the 3'-end of the PAM sequence which the CRISPR enzyme recognizes.

The on-target may be (N)_{5∼50}-PAM or PAM-(N)_{5∼50}, and in this case, N may be A, T, G or C; or A, U, G or C.

In this case, the on-target may be positioned in a genome in a cell.

The cell may be a prokaryotic cell or a eukaryotic cell.

In this case, the on-target may include a selection element.

The selection element is a toxin gene, an antibiotic resistance gene, a gene encoding fluorescent protein, a tagging gene, a lacZ gene or a gene encoding luciferase.

The toxin gene may be a Hok, fst, TisB, LdrD, FlmA, Ibs, TxpA/BrnT, SymE, XCV1262, CcdB, ParE, MazF, yafO, HicA, Kid, Zeta, DarT or Sxt gene, and is not limited thereto.

The toxin gene may be changed according to the source of the on-target (that is, according to the origin). That is, the toxin gene can be appropriately selected and used by the person skilled in the art according to the type of host cell.

When a host is *E. coli*(*Escherichia coli*), the toxin gene ccdB can be used.

When a host cell is a mammalian cell, the toxin gene hypoxanthine phosphoribosyl transferase (HPRT) can be used.

When the host is yeast, the toxin gene URA3 can be used.

The antibiotic resistance gene may be designed variously according to the type of antibiotic.

The antibiotic may be kanamycin, spectinomycin, streptomycin, ampicillin, carbenicillin, bleomycin, erythromycin, polymyxin B, tetracycline, zeocin, puromycin, or chloramphenicol, and is not limited thereto.

The fluorescent protein may be a green fluorescent protein (GFP), a red fluorescent protein (RFP), a yellow fluorescent protein (YFP), a cyan fluorescent protein (CFP), a blue fluorescent protein (BFP) or an orange fluorescent protein (OFP), and is not limited thereto.

The tag may be AviTag, Calmodulin-tag, polyglutamate tag, E-tag, FLAG-tag, HA-tag, His-tag, Myc-tag, NE-tag, S-tag, SBP-tag, Softag 1, Softag 3, Strep-tag, TC tag, V5 tag, VSV-tag, Xpress tag, Isopeptag, SpyTag, SnoopTag, Biotin Carboxyl Carrier Protein (BCCP), Glutathione-S-transferase (GST)-tag, HaloTag, Maltose binding protein (MBP)-tag, Nus-tag, Thioredoxin-tag, chitin binding protein (CBP), thioredoxin (TRX), or poly(NANP).

In this case, when an on-target including the selection element is cleaved or modified by a target specific nuclease, the selection element may not be expressed.

### 3-3-2 Off-target

The off-target may be a sequence or position of a non-target gene or nucleic acid to which a target specific nuclease partially complementarily binds.

The off-target may be a sequence of a nucleic acid including one or more other base sequences in the on-target.

The off-target may be a sequence or position of a gene or nucleic acid having a sequence partially complementary to a nucleic acid sequence of a part capable of specifically recognizing a target of a target specific nuclease.

When the target specific nuclease is a CRISPR-Cas system, the off-target may be a sequence or position of a gene or nucleic acid partially complementarily binding to a guide RNA or a guide sequence of the guide RNA.

In this case, the sequence of the gene or nucleic acid complementarily binding to the guide RNA or a guide sequence of the guide RNA may be 5 to 50 bps, and further, the length thereof may be adjusted according to the length of the nucleic acid sequence of the guide RNA or a guide sequence of the guide RNA.

When the target specific nuclease is a CRISPR-Cas system, the off-target may include a PAM sequence which a CRISPR enzyme recognizes.

When the target specific nuclease is a CRISPR-Cas system, the off-target may include a nucleic acid sequence complementarily binding to a guide RNA or a guide sequence of the guide RNA and a PAM sequence which a CRISPR enzyme recognizes.

In this case, the nucleic acid sequence complementarily binding to the guide RNA or a guide sequence of the guide RNA of the off-target may be positioned at the 5'-end of the PAM sequence which the CRISPR enzyme recognizes.

In this case, the nucleic acid sequence complementarily binding to the guide RNA or a guide sequence of the guide RNA of the off-target may be positioned at the 3'-end of the PAM sequence which the CRISPR enzyme recognizes.

The off-target may be (N)_{5∼50}-PAM or PAM-(N)_{5∼50}, and in this case, N may be A, T, G or C; or A, U, G or C.

The off-target may be a sequence or position of a gene or nucleic acid including one or more bonds non-complementary to a nucleic acid sequence of a part of specifically recognizing a target of a target specific nuclease.

The off-target may be a sequence or position of a gene or nucleic acid including bonds which are less than 100% complementary to a nucleic acid sequence of a part of specifically recognizing a target of a target specific nuclease. The nucleic acid sequence having a sequence homology less than 100% is a nucleic acid sequence similar to the nucleic acid sequence of the on-target, and may be a nucleic acid sequence in which one or more different base sequences are included or one or more base sequences are deleted.

When the target specific nuclease is a CRISPR-Cas system, the off-target may be a sequence or position of a gene or nucleic acid including one or more bonds non-complementary to the nucleic acid sequence of a guide RNA or a guide sequence of the guide RNA.

When the target specific nuclease is a CRISPR-Cas system, the off-target may be a sequence or position of a gene or nucleic acid including bonds which are less than 100% complementary to the nucleic acid sequence of a guide RNA or a guide sequence of the guide RNA.

When the target specific nuclease is a CRISPR-Cas system, the off-target may include one or more mismatched nucleic acid sequences among a PAM sequence which a CRISPR enzyme recognizes.

In this case, the off-target may be positioned in a genome in a cell.

The cell may be a prokaryotic cell or a eukaryotic cell.

In this case, the off-target may include a selection element.

The selection element is a toxin gene, an antibiotic resistance gene, a gene encoding fluorescent protein, a tagging gene, a lacZ gene or a gene encoding luciferase.

In this case, when an off-target including the selection element is cleaved or modified by a target specific nuclease, the selection element may not be expressed.

In this case, when an off-target including the selection element is not cleaved or modified by a target specific nuclease, the selection element may be expressed.

### 3-4 Effect of selection element

The term "selection element" of the present invention refers to a marker for detecting whether a target gene or nucleic acid sequence is cleaved or modified by a target specific nuclease. Therefore, the "selection element" can be used interchangeably with "a selection marker".

Based on whether the selection element is expressed or not, it is possible to detect whether a target gene or nucleic acid sequence is cleaved or modified by a target specific nuclease. That is, it is possible to detect whether the on-target effect is increased and/or whether the off-target effect is decreased.

A selection element included in an on-target and/or an off-target may be an element which determines high specificity or high activity of a target specific nuclease.

As an example, when a target specific nuclease is screened by using an on-target including a toxin gene as a selection element and an off-target including an antibiotic resistance gene as a selection element, if respective vectors including the on-target and the off-target and a vector including the target specific nuclease are introduced into a cell, it is possible to expect that the target specific nuclease exhibiting high specificity cleaves the on-target, and as a result, the expression of the toxin gene is suppressed, whereas the off-target is not cleaved, and as a result, the antibiotic resistance gene is expressed. Therefore, a cell where only the on-target is cleaved and the off-target is not cleaved, into which a target specific nuclease having high specificity is introduced, can survive when treated with an antibiotic. Accordingly, it is possible to screen a target specific nuclease having high specificity or high activity based on whether these selection elements are expressed or not.

As another example, when a target specific nuclease is screened by using an on-target including a toxin gene as a selection element and an off-target present in a genome of a cell, if a vector including the on-target and a vector including the target specific nuclease are introduced into a cell, it is possible to expect that the target specific nuclease exhibiting high specificity cleaves the on-target, and as a result, the expression of the toxin gene is suppressed, whereas the off-target is not cleaved, and as a result, the genome is not cleaved. Therefore, a cell where only the on-target is cleaved and the off-target is not cleaved, into which a target specific nuclease having high specificity is introduced, can survive, and a cell in which both the on-target and the off-target are cleaved cannot survive because the genome is cleaved due to the cleavage of the off-target, so that the two types of cells can be distinguished from each other.

As still another example, when a target specific nuclease is screened by using an on-target including a toxin gene as a selection element and an off-target including a gene encoding fluorescent protein as a selection element, if a vector including the on-target and a vector including the target specific nuclease are introduced into a cell, it is possible to expect that the target specific nuclease exhibiting high specificity cleaves the on-target, and as a result, the expression of the toxin gene is suppressed, whereas the off-target is not cleaved, and as a result, the fluorescent protein is expressed. Therefore, a cell where only the on-target is cleaved and the off-target is not cleaved, into which a target specific nuclease having high specificity is introduced, can survive, and can also be distinguished with a fluorescent protein.

Accordingly, a selection element included in an on-target and/or an off-target may be a reference for determining and selecting high specificity or high activity of a target specific nuclease.

### 4. Selection method of nuclease

A target specific nuclease having high specificity or high activity can be selected by using the selection system.

As an aspect, the present invention relates to a method for selecting a target specific nuclease having high specificity or high activity among a plurality of target specific nuclease test groups.

In the present invention, the "method for selecting a target specific nuclease" includes selecting a cell including a target specific nuclease having high specificity or high activity, and then identifying the constitution of a specific nuclease. For example, sequence information of an excellent target specific nuclease can be obtained by the present invention.

In particular, the method is very useful in selecting a target specific nuclease having high specificity or high activity among variants of various nucleases prepared by improving a target specific nuclease and a nuclease library.

### 4-1. First specific embodiment of selection method

As a specific embodiment,
the method can be utilized as a method for selecting a target specific nuclease having high specificity and high activity among various "variants" of a target specific nuclease.

The method is a method for selecting a target specific nuclease having high specificity or high activity, the method using:
i) multiple targets including one or more on-targets and one or more off-targets; and
ii) a target specific nuclease variant, the method including:
   a) introducing i) and ii) components into a cell;
   b) sorting the cell by identifying the on-target effect and the off-target effect based on whether a selection element is expressed or not in the a); and
   c) selecting the nuclease included in the sorted cell in the b) as the target specific nuclease having high specificity or high activity.

### 4-1-1 Components

In this case, i) component can use a vector system, and in i) component, the on-target and the off-target can be included in respective vectors.

A target specific nuclease of ii) component is a CRISPR-Cas system.

The CRISPR-Cas system consists of a guide RNA and a CRISPR enzyme.

A target specific nuclease variant of ii) component may be a variant of the CRISPR-Cas system, may be preferably a variant of the CRISPR enzyme, but is not limited thereto. The nuclease variant can be produced by electromagnetic waves, UV, radiation, chemicals, external/internal gene action, and the like. In an example, variants were obtained by irradiating a WT CRISPR-Cas system with UV.

The CRISPR enzyme may be Cas9, and a variant of the CRISPR enzyme may be a variant of Cas9.

The variant of Cas9 may be a variant in which an on-target effect is increased or decreased. In this case, the on-target effect means that an on-target position is cleaved or modified by Cas9.

The variant of Cas9 may be a variant in which an off-target effect is increased or decreased. In this case, the off-target effect means that an off-target position is cleaved or modified by Cas9.

Preferably, the variant of Cas9 may be a variant in which the on-target effect is increased and/or the off-target effect is decreased.
ii) Component can use a vector system, and when ii) component is a CRISPR-Cas system, the guide RNA and the CRISPR enzyme may be included in the same vector or different vectors.
ii) Component can use a ribonucleoprotein (RNP) system, and when ii) component is a CRISPR-Cas system, the guide RNA and the CRISPR enzyme may be in the form of a RNA-protein complex.
ii) Component may be one artificially synthesized.

When ii) component is a CRISPR-Cas system, the guide RNA may be one artificially synthesized, and the CRISPR enzyme may also be a protein or polypeptide artificially synthesized.

In i) component, the on-target may be a sequence or position of a gene or nucleic acid complementarily binding to a guide RNA or a guide sequence of the guide RNA of ii) component.

In i) component, the off-target may be a sequence or position of a gene or nucleic acid partially complementarily binding to a guide RNA or a guide sequence of the guide RNA of ii) component.

In i) component, the off-target may be a sequence or position of a gene or nucleic acid forming one or more bonds non-complementary to the nucleic acid sequence of a guide RNA or a guide sequence of the guide RNA of ii) component.

In i) component, the on-target and/or the off-target may include a selection element.

The selection element is a toxin gene, an antibiotic resistance gene, a gene encoding fluorescent protein, a tagging gene, a lacZ gene or a gene encoding luciferase.

### 4-1-2 Method

In this case, when i) and ii) components are introduced into a cell in the a), i) and ii) components can be introduced into the cell by transfection using a virus vector system, a ribonucleoprotein (RNP), nanoparticles, a liposome, and the like, microinjection, electroporation, and the like, but the introduction method is not limited thereto.

The cell may be a prokaryotic cell or a eukaryotic cell.

Meanwhile, the off-target may be present in a genome of a cell.

When the off-target among i) component is present in the cell genome, a vector including the on-target of i) component and ii) component can be introduced into the cell.

Meanwhile, the on-target may be present in a genome of a cell.

When the on-target among i) component is present in the cell genome, a vector including the off-target of i) component and ii) component can be introduced into the cell.

In this case, in the b),
among cells into which i) and ii) components are introduced in the a), a cell in which only the on-target among i) component is cleaved by ii) component can be selected.

The cell in which only the on-target is cleaved can be selected by the suppression of expression of a selection element included in the on-target.

For example, when the on-target includes a toxin gene, the cell can survive due to the suppression of expression of the toxin gene as the on-target is cleaved.

Further, the off-target among i) component can select a cell which is not cleaved by ii) component.

The cell in which the off-target is not cleaved can be selected by the expression of the selection element because the off-target is not cleaved.

For example, when the off-target includes an antibiotic resistance gene, a cell having antibiotic resistance can be selected in the presence of an antibiotic through the expression of the antibiotic resistance gene as the off-target is not cleaved.

Alternatively, when the off-target is positioned in a genome in a cell, the genome of the cell is not cleaved by ii) component, so that the genome of the cell is normally maintained, and as a result, the cell can survive.

In this case, the sorted cell in the b) may be a cell in which the on-target of i) component is cleaved and the off-target is not cleaved by ii) component. These cells can be distinguished by selection elements.

In this case, in the c),

it is possible to identify ii) component introduced into the sorted cell, that is, a target specific nuclease by using the sorted cell in the b).
ii) Component introduced into the sorted cell, that is, a target specific nuclease by using the sorted cell in the b) may be a target specific nuclease capable of selectively cleaving or modifying only the on-target.

A sequence of the nuclease can be acquired by sequencing the nuclease obtained from the sorted cell in the c), thereby obtaining a target specific nuclease having high specificity or high activity.

For example, when colonies are produced to sizes which can be seen by the unaided eye by culturing *E. coli(Escherichia coli)* subjected to electroporation in an LB agar plate including kanamycin, chloramphenicol, and arabinose at 30°C for 16 hours, each one of the colonies is inoculated into 10 ml of a LB buffer including chloramphenicol, and then cultured in a shaking incubator at 42°C for 12 hours. After *E. coli* pellets are obtained through centrifugation, a plasmid is extracted from the pellets by using a miniprep kit. The extracted plasmid can be sequenced by using, for example, a Sanger sequencing method. In the case of a primer, it is possible to use a universal sequencing primer such as CMV-F and BGH-R present outside of the Cas 9 N-term and C-term and sequencing primers in the middle part of Cas 9.

As a specific embodiment of the present invention, when described with reference to FIG. 1, in the method for identifying a target specific nuclease, all of Plasmids A, B, and C were introduced in an LB agar plate including kanamycin and chloramphenicol which are identification markers of Plasmids B and C and arabinose operating an arabinose promoter through electroporation, when *E. coli* incubated in an SOC including anhydrous tetracycline for 1 hour was aliquoted, Plasmid A was cleaved by a nuclease of Plasmid C, but only the *E*. *coli,* whose genomic DNA was not cleaved, survived.

Accordingly, a target specific nuclease having high specificity or high activity can be selected through the sorted cell by using the method as described above.

### 4-2. Second specific embodiment of selection method

As another specific embodiment,

the method can be utilized as a method for selecting a target specific nuclease having high specificity and high activity in a target specific nuclease "library".

The method is a method for selecting a target specific nuclease having high specificity or high activity, the method using:
i) multiple targets including one or more on-targets and one or more off-targets; and
ii) a target specific nuclease library, the method including:

a) introducing i) and ii) components into a cell;
b) sorting a cell in which only the on-target is modified in the a); and
c) identifying ii) component by using the selected cell in the b).

### 4-2-1 Components

In this case, i) component can use a vector system, and in i) component, the on-target and the off-target can be included in respective vectors.

A target specific nuclease of ii) component is a CRISPR-Cas system.

The target specific nuclease library may be a CRISPR-Cas system library. The nuclease library may be directly manufactured, may also be commercially available, and may also be obtained from a database.

The CRISPR-Cas system consists of a guide RNA and a CRISPR enzyme.

The CRISPR-Cas system library may be a guide RNA library and/or a CRISPR enzyme library.
ii) Component can use a vector system, and when ii) component is a CRISPR-Cas system, the guide RNA and the CRISPR enzyme may be included in the same vector or different vectors.
ii) Component can use a ribonucleoprotein (RNP) system, and when ii) component is a CRISPR-Cas system, the guide RNA and the CRISPR enzyme may be in the form of a RNA-protein complex.
ii) Component may be one artificially synthesized.

In i) component, the on-target may be a sequence or position of a gene or nucleic acid complementarily binding to a guide RNA or a guide sequence of the guide RNA of ii) component.

In i) component, the off-target may be a sequence or position of a gene or nucleic acid partially complementarily binding to a guide RNA or a guide sequence of the guide RNA of ii) component.

In i) component, the off-target may be a sequence or position of a gene or nucleic acid forming one or more bonds non-complementary to a guide RNA or a guide sequence of the guide RNA of ii) component.

In i) component, the on-target and/or the off-target may include a selection element.

The selection element may be a toxin gene, an antibiotic resistance gene, a gene encoding fluorescent protein, a tagging gene, a lacZ gene or a gene encoding luciferase, and is not limited thereto.

### 4-2-2 Method

In this case, when i) and ii) components are introduced into a cell in the a), i) and ii) components can be introduced into the cell by transfection using a virus vector system, a ribonucleoprotein (RNP), nanoparticles, a liposome, and the like, microinjection, electroporation, and the like, but the introduction method is not limited thereto.

The cell may be a prokaryotic cell or a eukaryotic cell.

Meanwhile, the off-target may be present in a genome of a cell.

When the off-target among i) component is present in the cell genome, a vector including the on-target of i) component and ii) component can be introduced into the cell.

Meanwhile, the on-target may be present in a genome of a cell.

When the on-target among i) component is present in the cell genome, a vector including the off-target of i) component and ii) component can be introduced into the cell.

In the b),
among cells into which i) and ii) components are introduced in the a), a cell in which only the on-target among i) component is cleaved by ii) component can be selected.

The cell in which only the on-target is cleaved can be selected by the suppression of expression of a selection element included in the on-target.

For example, when the on-target includes a toxin gene, the cell can survive due to the suppression of expression of the toxin gene as the on-target is cleaved.

Further, the off-target among i) component can select a cell which is not cleaved by ii) component.

The cell in which the off-target is not cleaved can be selected by the expression of the selection element because the off-target is not cleaved.

For example, when the off-target includes an antibiotic resistance gene, a cell having antibiotic resistance can be selected in the presence of an antibiotic through the expression of the antibiotic resistance gene as the off-target is not cleaved.

Alternatively, when the off-target is positioned in a genome in a cell, the genome of the cell is not cleaved by ii) component, so that the genome of the cell is normally maintained, and as a result, the cell can survive.

In this case, the sorted cell in the b) may be a cell in which the on-target of i) component is cleaved and the off-target is not cleaved by ii) component. These cells can be distinguished by selection elements.

In this case, in the c),

it is possible to identify ii) component introduced into the sorted cell, that is, a target specific nuclease by using the sorted cell in the b).
ii) Component introduced into the sorted cell, that is, a target specific nuclease by using the sorted cell in the b) may be a target specific nuclease capable of selectively cleaving or modifying only the on-target.

A sequence of the nuclease can be acquired by sequencing the nuclease obtained from the sorted cell in the c), thereby obtaining a target specific nuclease having high specificity or high activity.

For example, when colonies are produced to sizes which can be seen by the unaided eye by culturing *E. coli(Escherichia coli)* subjected to electroporation in an LB agar plate including kanamycin, chloramphenicol, and arabinose at 30°C for 16 hours, each one of the colonies is inoculated into 10 ml of a LB buffer including chloramphenicol, and then cultured in a shaking incubator at 42°C for 12 hours. After *E. coli* pellets are obtained through centrifugation, a plasmid is extracted from the pellets by using a miniprep kit. The extracted plasmid can be sequenced by using, for example, a Sanger sequencing method. In the case of a primer, it is possible to use a universal sequencing primer such as CMV-F and BGH-R present outside of the Cas 9 N-term and C-term and sequencing primers in the middle part of Cas 9.

As a specific embodiment of the present invention, when described with reference to FIG. 1, in the method for identifying a target specific nuclease, all of Plasmids A, B, and C were introduced in an LB agar plate including kanamycin and chloramphenicol which are identification markers of Plasmids B and C and arabinose operating an arabinose promoter through electroporation, when *E. coli* incubated in an SOC including anhydrous tetracycline for 1 hour was aliquoted, Plasmid A was cleaved by a nuclease of Plasmid C, but only the *E*. *coli,* whose genomic DNA was not cleaved, survived.

Accordingly, it is possible to select a target specific nuclease having high specificity or high activity in a target specific nuclease library through the sorted cell by using the method as described above.

### 5. Composition or Kit

Another aspect of the present invention relates to a screening kit for selecting a target specific nuclease having high specificity or high activity, including: multiple targets containing one or more selection elements. The kit may be in the form of a composition.

In a specific embodiment, the composition or kit may consist of:
i) multiple targets containing one or more selection elements; and
ii) a target specific nuclease and/or iii) a host cell.

The screening kit or composition for selecting a target specific nuclease having high specificity or high activity according to the present invention includes multiple targets containing one or more selection elements as an essential component, and selectively includes a target specific nuclease and/or a host cell.

If necessary, the kit of the present invention may be provided as a one liquid-type kit including multiple targets containing one or more selection elements.

If necessary, the kit of the present invention may be provided as a two liquid-type kit respectively including multiple targets containing one or more selection elements and a host cell.

In this case, the kit may also be provided as a one liquid-type by including multiple targets including a selection element in a host cell. For example, the kit may also be included as a separate plasmid in a host cell, and may also be provided by incorporation into a host cell genome.

If necessary, the kit of the present invention may be provided as a three liquid-type kit respectively including multiple targets containing one or more selection elements, a host cell, and a test nuclease.

In this case, the kit may also be provided as a two liquid-type by including multiple targets including a selection element in a host cell. For example, the kit may also be included as a separate plasmid in a host cell, and may also be provided by incorporation into a host cell genome.

In this case, i) component can use a vector system, and in i) component, one or more on-targets and one or more off-targets can be included in respective vectors.

A target specific nuclease of ii) component is a CRISPR-Cas system.

The CRISPR-Cas system consists of a guide RNA and a CRISPR enzyme.
ii) Component can use a vector system, and when ii) component is a CRISPR-Cas system, the guide RNA and the CRISPR enzyme may be included in the same vector or different vectors.
ii) Component can use a ribonucleoprotein (RNP) system, and when ii) component is a CRISPR-Cas system, the guide RNA and the CRISPR enzyme may be in the form of a RNA-protein complex.
ii) Component may be one artificially synthesized.

When ii) component is a CRISPR-Cas system, the guide RNA may be one artificially synthesized, and the CRISPR enzyme may also be a protein or polypeptide artificially synthesized.

In i) component, the on-target may be a sequence or position of a gene or nucleic acid complementarily binding to a guide RNA or a guide sequence of the guide RNA of ii) component.

In i) component, the off-target may be a sequence or position of a gene or nucleic acid partially complementarily binding to a guide RNA or a guide sequence of the guide RNA of ii) component.

In i) component, the off-target may be a sequence or position of a gene or nucleic acid forming one or more bonds non-complementary to the nucleic acid sequence of a guide RNA or a guide sequence of the guide RNA of ii) component.

In i) component, the on-target and/or the off-target may be connected to a selection element.

The selection element may be a toxin gene, an antibiotic resistance gene, a gene encoding fluorescent protein, a tagging gene, a lacZ gene or a gene encoding luciferase, and is not limited thereto.
iii) Component host cell may be any cell suitable for the expression of the selection element. iii) Component host cell may be a prokaryotic cell or a eukaryotic cell.

For example, *E. coli* can be used as a host when the toxin gene ccdB is used, a cell derived from a mammal can be used as a host cell when a hypoxanthine phosphoribosyl transferase (HPRT) is used, and yeast can be used as a host when the toxin gene URA3 is used.

Moreover, it will be obvious that those skilled in the art of the present invention can arbitrarily select and use a type of cell suitable for the screening and selection of a nuclease.

Furthermore, the composition or kit may additionally include a buffer, various materials (for example, antibiotics, X-gal, and the like) for selection or various reagents (for example, a transfection reagent, lipofectamine, and the like) used for introduction in a cell, if necessary.

### 6. Cell (line) or Method for producing cell

Further, the present disclosure provides a cell including multiple targets for screening and/or selecting a target specific nuclease and/or a method for producing the cell.

The cell is characterized by including multiple targets.

In this case, the multiple targets may be those present in a genome in a cell or introduced into the cell from exogenous sources.

The cell may be a prokaryotic cell or a eukaryotic cell.

### 6-1 Multiple targets

The target may be a gene or nucleic acid sequence targeted by a target specific nuclease.

The multiple targets may consist of a gene or nucleic acid sequence to be targeted (that is, an on-target) and a gene or nucleic acid sequence which is not targeted (that is, an off-target).

The multiple targets may consist of one or more on-targets and one or more off-targets.

When the multiple targets are introduced into a cell from exogenous sources, the multiple targets may be introduced by using a vector system.

When the multiple targets are introduced into a cell from exogenous sources, the multiple targets can be introduced by using a different vector for each target. That is, when the multiple targets consist of one on-target and one off-target, the vector may consist of a vector including an on-target and a vector including an off-target.

### 6-1-1 On-target

The on-target may be a sequence or position of a target gene or nucleic acid to which a target specific nuclease complementarily binds.

The on-target may be a sequence or position of a gene or nucleic acid having a sequence complementary to a nucleic acid sequence of a part capable of specifically recognizing a target of a target specific nuclease.

When the target specific nuclease is a CRISPR-Cas system, the on-target may be a sequence or position of a gene or nucleic acid complementarily binding to a guide RNA or a guide sequence of the guide RNA.

In this case, the sequence of the gene or nucleic acid complementarily binding to the guide RNA or a guide sequence of the guide RNA may be 5 to 50 bps, and further, the length thereof may be adjusted according to the length of the nucleic acid sequence of the guide RNA or a guide sequence of the guide RNA.

When the target specific nuclease is a CRISPR-Cas system, the on-target may include a PAM sequence which a CRISPR enzyme recognizes.

When the target specific nuclease is a CRISPR-Cas system, the on-target may include a nucleic acid sequence complementarily binding to a guide RNA or a guide sequence of the guide RNA and a PAM sequence which a CRISPR enzyme recognizes.

In this case, the nucleic acid sequence complementarily binding to the guide RNA or a guide sequence of the guide RNA of the on-target may be positioned at the 5'-end of the PAM sequence which the CRISPR enzyme recognizes.

In this case, the nucleic acid sequence complementarily binding to the guide RNA or a guide sequence of the guide RNA of the on-target may be positioned at the 3'-end of the PAM sequence which the CRISPR enzyme recognizes.

The on-target may be (N)_{5∼50}-PAM or PAM-(N)_{5∼50}, and in this case, N may be A, T, G or C; or A, U, G or C.

In this case, the on-target may be positioned in a genome in a cell.

In this case, the on-target may include a selection element.

The selection element is a toxin gene, an antibiotic resistance gene, a gene encoding fluorescent protein, a tagging gene, a lacZ gene or a gene encoding luciferase.

When an on-target including the selection element is cleaved or modified by a target specific nuclease, the selection element may not be expressed.

### 6-1-2 Off-target

The off-target may be a sequence or position of a non-target gene or nucleic acid to which a target specific nuclease partially complementarily binds.

The off-target may be a sequence of a nucleic acid including one or more other base sequences in the on-target.

The off-target may be a sequence or position of a gene or nucleic acid having a sequence partially complementary to a nucleic acid sequence of a part capable of specifically recognizing a target of a target specific nuclease.

When the target specific nuclease is a CRISPR-Cas system, the off-target may be a sequence or position of a gene or nucleic acid partially complementarily binding to a guide RNA or a guide sequence of the guide RNA.

In this case, the sequence of the gene or nucleic acid complementarily binding to the guide RNA or a guide sequence of the guide RNA may be 5 to 50 bps, and further, the length thereof may be adjusted according to the length of the nucleic acid sequence of the guide RNA or a guide sequence of the guide RNA.

When the target specific nuclease is a CRISPR-Cas system, the off-target may include a PAM sequence which a CRISPR enzyme recognizes.

When the target specific nuclease is a CRISPR-Cas system, the off-target may include a nucleic acid sequence complementarily binding to a guide RNA or a guide sequence of the guide RNA and a PAM sequence which a CRISPR enzyme recognizes.

In this case, the nucleic acid sequence complementarily binding to the guide RNA or a guide sequence of the guide RNA of the off-target may be positioned at the 5'-end of the PAM sequence which the CRISPR enzyme recognizes.

In this case, the nucleic acid sequence complementarily binding to the guide RNA or a guide sequence of the guide RNA of the off-target may be positioned at the 3'-end of the PAM sequence which the CRISPR enzyme recognizes.

The off-target may be (N)_{5∼50}-PAM or PAM-(N)_{5∼50}, and in this case, N may be A, T, G or C; or A, U, G or C.

The off-target may be a sequence or position of a gene or nucleic acid including one or more bonds non-complementary to a nucleic acid sequence of a part of specifically recognizing a target of a target specific nuclease.

The off-target may be a sequence or position of a gene or nucleic acid including bonds which are less than 100% complementary to a nucleic acid sequence of a part of specifically recognizing a target of a target specific nuclease.

When the target specific nuclease is a CRISPR-Cas system, the off-target may be a sequence or position of a gene or nucleic acid including one or more bonds non-complementary to the nucleic acid sequence of a guide RNA or a guide sequence of the guide RNA.

When the target specific nuclease is a CRISPR-Cas system, the off-target may be a sequence or position of a gene or nucleic acid including bonds which are less than 100% complementary to the nucleic acid sequence of a guide RNA or a guide sequence of the guide RNA.

When the target specific nuclease is a CRISPR-Cas system, the off-target may include one or more mismatched nucleic acid sequences among a PAM sequence which a CRISPR enzyme recognizes.

In this case, the off-target may be positioned in a genome in a cell.

The off-target may additionally include a selection element.

The selection element is a toxin gene, an antibiotic resistance gene, a gene encoding a fluorescent protein, a tagging gene, a lacZ gene or a gene encoding luciferase.

In this case, when an off-target including the selection element is cleaved or modified by a target specific nuclease, the selection element may not be expressed.

In this case, when an off-target including the selection element is not cleaved or modified by a target specific nuclease, the selection element may be expressed.

The cell may include multiple targets variously designed according to the target of the target specific nuclease.

In addition, the cell may include only some targets among the multiple targets.

That is, the cell may include only the on-target or only the off-target among the multiple targets.

### 6-2 Production method

The method for producing the cell can use a method for introducing a vector system including multiple targets into a cell.

The introduction of the multiple targets into the cell can be carried out by transfection using a virus vector system, nanoparticles, a liposome, and the like, microinjection, electroporation, and the like, but the introduction method is not limited thereto.

Further, the introduction of the multiple targets into the cell can simultaneously introduce the multiple targets.

That is, it is possible to simultaneously introduce a vector including an on-target and a vector including an off-target, which are the multiple targets, into the cell.

The introduction of the multiple targets into the cell can separately introduce the multiple targets.

That is, it is possible to introduce a vector including an off-target after firstly introducing a vector including an on-target into the cell, among the multiple targets. Alternatively, it is possible to introduce a vector including an on-target after firstly introducing a vector including an off-target into the cell, among the multiple targets.

Further, the introduction of the multiple targets into the cell can introduce only some targets among the multiple targets.

That is, it is possible to introduce only a vector including an on-target in the multiple targets into a cell. Alternatively, it is possible to introduce only a vector including an off-target in the multiple targets into a cell.

The system of the present invention can be usefully utilized in selecting a target specific nuclease having high specificity and high activity among any nuclease test group. Furthermore, a nuclease selected by using the system of the present invention can decrease an off-target effect and increase an on-target effect when a gene is corrected or manipulated.

### Example

Hereinafter, the present invention will be described in more detail through Examples.

These Examples are only for exemplifying the present invention, and it will be obvious to a person with ordinary skill in the art that the scope of the present invention is not interpreted to be limited by these Examples.

### Example 1. Screening of Cas9 Variants in E. coli

### 1-1 Control Experiment

CATCAACATCGAATACATGA NAG (SEQ ID No. 1) is a repeated sequence found in K12 *E. coli* genomic DNA. The sequence includes a PAM sequence of NAG similar to NGG, and thus is known to be cleaved with low efficiency by a CRISPR-CAS9 enzyme.

CATCAACATCGAATACATGA (SEQ ID No. 2), which is a 20-mer guide sequence from the sequence, was used as a sgRNA in Plasmid B. CATCAACATCGAATACATGA TGG (SEQ ID No. 3), a 23-mer target sequence, targeted by the sgRNA was inserted as a target site into Plasmid A. The target sequence has NGG, which is expected to exhibit high cleavage efficiency instead of NAG, as a PAM site.

Plasmids A and B were both introduced into BW25141 *E. coli* by using electroporation. The cells, into which Plasmids A and B were introduced, were cultured under a condition of 37°C in an LB medium including ampicillin and kanamycin antibiotics. After a colony was grown through the cell culture, an electrocompetent cell was prepared from the colony by using a standard method.

Plasmid C including WT CAS9 as a positive control or a null vector as a negative control was introduced into the electrocompetent cell including Plasmids A and B. After the cells were cultured in a SOC medium for 1 hour, half of the cultured cells were cultured in an LB medium including chloramphenicol, and the other half of the cultured cells were cultured in an LB medium including chloramphenicol, kanamycin, 10 mM arabinose, and 100 ng/ml of anhydrotetracycline (ATC).

As a result, about 10,000 colonies of all the cells into which Plasmid C and the null vector were each introduced could be identified in the LB medium including chloramphenicol, whereas no colony was found in the LB medium including chloramphenicol, kanamycin, arabinose, and anhydrotetracycline. The above result is described below.

Since expressed WT CAS9 had residual activity against NAG which is the replaced PAM sequence in the cells into which Plasmid C including WT CAS9 was introduced, the *E*. *coli* genomic DNA sequence including NAG was cleaved. On the other hand, since Plasmid A including a toxin gene could not be cleaved in the cells into which Plasmid C (that is, a null vector) including nothing was introduced due to the absence of cleavage activity, the expression of the toxin gene was induced in the LB medium including arabinose, thereby showing apoptosis.

For another control, Plasmids A' and B' including ctagatgagaccggatccggtctccTGG (SEQ ID No. 4) and ctagatgagaccggatccggtctcc (SEQ ID No. 5) which are sequences not present in *E. coli* genomic DNA, respectively, were used as a positive control. After Plasmid C including WT CAS9 was introduced thereinto, about 10,000 colonies were each observed in both the LB medium including only chloramphenicol and the LB medium including chloramphenicol, kanamycin, arabinose, and anhydrotetracycline. This is considered to be a result of WT CAS9 efficiently cleaving Plasmid A while not impairing the genomic DNA.

### 1-2 Mutation

After the successful control experiment, a random mutation by PCR amplification of a sequence of WT CAS9 was introduced by using a Genemorph II kit manufactured by Agilent Technologies, Inc. using an experimental method with a low error rate. PCR products were ligated into a backbone of doubly cleaved Plasmid C. A library with a size of 1,000,000 was constructed by introducing the plasmid produced by the ligation into a high-efficiency electrocomponent cell. The random library was introduced into the electrocomponent cell including the previously prepared Plasmids A and B.

Thereafter, the cell was cultured in two media in the same manner as in the previous experiment. As a result, about 2,500 colonies were identified in the LB medium including only chloramphenicol, whereas 180 colonies were exhibited in the LB medium including chloramphenicol, kanamycin, arabinose, and anhydrotetracycline. Each colony was cultured in the LB medium including chloramphenicol to express Plasmid C including a random mutation. The entire sequences of the random mutations obtained from the cultured colony had activity against NGG which is a general PAM sequence, whereas the entire sequences were identified as an effective mutation showing inhibited activity against NAG, a PAM sequence which is similar but is not general.

Through the experiment, a Cas9 variant having specificity for a guide sequence could be found. In this case, a mismatched sequence for the sequence of the sgRNA may be an *E*. *coli* endogenous genomic DNA sequence or an artificial sequence introduced and inserted into *E. coli* from exogenous sources.

By using the screening method described above, a plurality of Cas9 variants having activity against NGG which is a general PAM sequence, while having decreased activity against NAG, a PAM sequence which is similar but not general, was successfully identified.

### Example 2: Selection of Nuclease

It was confirmed that a 20-mer sgRNA having one mismatch with respect to a guide sequence of a target position in a WT EMX1 gene had low activity as compared to the case where a sgRNA having no mismatch, that is, being completely matched, was used in a previous study. The WT EMX1 gene is present in the genomic DNA of a mammalian cell, and PCR products obtained by amplifying a 500-mer base sequence including the WT EMX1 gene in the genomic DNA of the mammalian cell were inserted into the genomic DNA of *E*. *coli* (BW25141) by using a standard method (McKenzie GJ et al., BMC Microbiol. 2006, 6, 39). As a result, an obtained strain was named BW25141-EMX1, and then used.

A 20-mer guide sequence including one or two mismatch(es) with respect to gagtccgagcagaagaagaaggg (SEQ ID No. 6) which is a target sequence:
In the Example of the present invention,
56: gagtccgagcagaaAGagaa (SEQ ID No. 7);
1718: gaACccgagcagaagaagaa (SEQ ID No. 8);
7: gagtccgagcagaGgaagaa (SEQ ID No. 9); and
17: gagCccgagcagaagaagaa (SEQ ID No. 10)) was used as a sgRNA in Plasmid B.

A 23-mer target sequence (that is, 56(gagtccgagcagaGgaagaa ggg), 1718(gaACccgagcagaagaagaa ggg), 7(gagtccgagcagaGgaagaa ggg), 17(gagCccgagcagaagaagaa ggg)) corresponding to the sgRNA was inserted into Plasmid A as a target position.

Plasmids A and B produced as described above were both introduced into a BW25141-EMX1 *E. coli* strain by using electroporation. The cells, into which Plasmids A and B were introduced, were cultured under a condition of 37°C in an LB medium including ampicillin and kanamycin antibiotics. After a colony was cultured, an electrocomponent cell was manufactured by using a standard protocol. After a colony was grown through the cell culture, an electrocompetent cell was prepared from the colony by using a standard method.

### i) Control reaction of 56 and 1718

For a control reaction, Plasmid C including WT CAS9 or a null vector as a negative control was introduced into the electrocomponent cell including Plasmids A and B. After the cells, into which Plasmid C or the null vector was introduced, were cultured in an SOC medium including 1,000 ng/ml of anhydrous tetracycline for 1 hour, half of the cultured cells were cultured in an LB including chloramphenicol, and the other half of the cultured cells were cultured in an LB medium including chloramphenicol, kanamycin, 10 mM arabinose, and 1,000 ng/ml of anhydrous tetracycline.

A result obtained by carrying out a control experiment for Plasmid B including two mismatch sequences with respect to the EMX-1 sequence inserted into the genomic DNA of *E. coli,* that is, Plasmid B including 56 and Plasmid B including 1718 showed a comparison between the number of colonies formed per unit in an LB agar medium (CK) including chloramphenicol and kanamycin and the number of colonies formed per unit in an LB agar medium (CKA-1,000 ng/ml ATC) including chloramphenicol, kanamycin, arabinose, and 1,000 ng/ml of anhydrous tetracycline (FIG. 2).

When two mismatches were located near a seed region (56-WT-CAS9), less than 0.1% of the *E. coli* into which Cas9 was introduced survived. On the other hand, when a null vector, which is a negative control, was introduced into *E. coli* instead of Cas9, approximately 0.01% of *E. coli* survived. The result of the negative control is regarded as a background due to an effect of Plasmid A not acting.

### ii) Control reaction of 7 and 17

For a control reaction, Plasmid C including WT CAS9 or a null vector as a negative control was introduced into the electrocomponent cell including Plasmids A and B. After the cells, into which Plasmid C or the null vector was introduced, were cultured in an SOC medium including 10 ng/ml of anhydrous tetracycline for 1 hour, half of the cultured cells were cultured in an LB including chloramphenicol, and the other half of the cultured cells were cultured in an LB medium including chloramphenicol, kanamycin, 10 mM arabinose, and 10 ng/ml of anhydrous tetracycline.

A result obtained by carrying out a control experiment for Plasmid B including one mismatch sequence with respect to the EMX-1 sequence introduced into the genomic DNA of *E. coli,* that is, Plasmid B including 7 and Plasmid B including 17 showed a comparison between the number of colonies formed per unit in an LB agar medium (CK) including chloramphenicol and kanamycin and the number of colonies formed per unit in an LB agar medium (CKA-10 ng/ml ATC) including chloramphenicol, kanamycin, arabinose, and 10 ng/ml of anhydrous tetracycline (FIG. 3).

When one mismatch was located near a seed region (7-WT-CAS9), less than 0.1% of the *E. coli* into which Cas9 was introduced survived. On the other hand, when a null vector, which is a negative control, was introduced into *E. coli* instead of Cas9, approximately 0.01% of *E. coli* survived. The result of the negative control is regarded as a background due to an effect of Plasmid A not acting.

### iii) Screening of 56 and 1718

After the successful control experiment, a random mutation was introduced by three different means.
1. PCR Amplification of WT CAS9 Sequence Using Genemorph II Kit manufactured by Agilent Technologies, Inc.
2. Diversify PCR Random Mutagenesis Kit manufactured by Clontech Laboratories, Inc.
3. Mutagenic *E. coli* Strain (XL 1-red) Using Standard Experimental Method Having Low Error Rate

Each PCR product obtained by the method was ligated into a backbone of doubly cleaved Plasmid C. A library with a size of 2,000,000 was each constructed by introducing the plasmid produced by the ligation into a high-efficiency electrocomponent cell. Each of the random libraries was introduced into the electrocomponent cell prepared with BW25141-EMX1 including Plasmid A (including 56) and Plasmid B (including 56) previously prepared.

The cells, into which each random library was introduced, were cultured in a medium (CKA) including chloramphenicol, kanamycin, and arabinose and formed colonies, and an integrated library was formed by counting the number of colonies formed and collecting the colonies formed.

The obtained library was cultured under a condition of 42°C in an LB medium including chloramphenicol in order to selectively remove a sgRNA vector including an origin sensitive to the temperature. After the culture, the library was purified by using a miniprep in order to obtain a library vector.

The same experiment was performed on a library vector obtained through the purification by using BW25141-EMX1 including Plasmid A (including 1718) and Plasmid B (including 1718) in the same manner as described above for another selection.

The selection of the library vector was continuously performed until the ratio of CKA/CK colonies reached 100%.

### iv) Shuffling reaction

The library obtained through the selection was used in a shuffling reaction by using a standard shuffling method including an additional modification. Usually, when a PCR product is larger than 1 kb, it is known that the efficiency of shuffling significantly deteriorates. Therefore, a shuffling reaction for a Cas9 vector having a length of 4,300 bps does not easily occur.

The Cas9 vector was divided into 5 fragments, and each PCR fragment was shuffled by using a standard method. And then, the shuffling mixture was put together, and the PCR reaction was again performed. The PCR products obtained thereby were amplified by using a primer targeting the 5' end and the 3' end of Cas9, and it was confirmed from the amplified PCR products that a shuffling library was produced by identifying the size of the product using an agarose gel.

### v) Screening of 7 and 17

By using EMX1-BW25141 including Plasmid A (including 7 or 17) and Plasmid B (including 7 or 17) in a manner similar to the screening reaction using 56 and 1718, a shuffling library and the random mutation library prepared by using the Agilent and Clontech kits were repeatedly screened. The screening reaction was repeated until a stable ratio was reached. As a result, an obtained colony was analyzed by using a standard sequencing method.

### vi) Verification

By using a library in a full state obtained by simultaneously carrying out the two methods (a shuffling+1-mismatch (7 or 17) screening through a 1-mismatch (7 or 17) screening and a 2-mismatch (56 or 1718) screening), on-target and off-target experiments were carried out on each of a DMD gene and an EMX1 gene.

As a result, when a screening began with the 2-mismatch (56 or 1718), it could be confirmed that the off-target effect was more definitely decreased as compared to the WT Cas9 (FIGS. 6 and 7).

An experiment of identifying improved specificity in a mammalian cell was carried out by using three clones selected through the screening. On-target and off-target experiments were carried out on each of the DMD gene and the EMX1 gene.

As a result of determining on-target and off-target activities of the DMD gene by using a library in a full state obtained by the screening performed simultaneously by the two methods, it could be confirmed that when the screening began with 2 mismatches, the off-target activity was more definitely decreased as compared to the WT Cas9 (FIG. 8).

As a result of determining on-target and off-target activities of the EMX1 gene by using a library in a full state obtained by the screening performed simultaneously by the two methods, it could be confirmed that when the screening began with 2 mismatches, the off-target activity was more definitely decreased as compared to the WT Cas9 (FIG. 9).

Through these results, it was confirmed that both the two genes had improved specificity as compared to the WT-spCas9.

### Example 3: Screening of Mammalian cell

The experiment as described in Examples 1 and 2 can be carried out by using a mammalian cell.

A HPRT gene may be used like the toxin gene ccdB. A sequence of gRNA having a mismatch with HPRT is bound to the 5' end of a thymidine kinase and a cysteine deaminase (after the initiation codon), an entire sequence including a selection marker such as zeocin or puromycin can be inserted into a mammalian cell such as a HELA cell by using a standard method, and a stable cell line can be prepared by sorting a cell obtained therefrom using an antibiotic.

A vector including a Cas9 variant can be inserted into the genomic DNA of the cell line by a standard method using a transposon (in this case, includes a transposase, and may be, for example, Piggybac, sleepingbeauty, and the like) or a virus (lentivirus). A sgRNA vector targeting HPRT can be transfected into the cell line, and HPRT and TK or CD can be each selected by using 6-thioguanine and ganciclovir or 5-fluorocytosine.

The thus selected colony includes a Cas9 variant with improved specificity. The screening method can be used without TK or CD, and a sgRNA showing negligible activity can be used in order to find a Cas9 having increased activity.

### [Industrial Applicability]

The system of the present invention can be usefully utilized in selecting a target specific nuclease having high specificity and high activity among any nuclease test group. Furthermore, a nuclease selected by using the system of the present invention may decrease an off-target effect and increase an on-target effect when a gene is corrected or manipulated.
## Claims

1. A method for evaluating a specificity or an activity of a CRISPR enzyme, the method comprising:
providing at least one cell including a first plasmid and a genome,
wherein the first plasmid including,
a first nucleic acid, and
a first selection element being operably linked to the first nucleic acid,
wherein the first selection element is a gene selected from the group consists of a toxin gene, an antibiotic resistance gene, a gene encoding fluorescent protein, a tagging gene, a lacZ gene and a gene encoding luciferase,
wherein the genome including a second nucleic acid having a sequence homology less than 100% with the first nucleic acid,
contacting a CRISPR complex to the first nucleic acid and the second nucleic acid respectively,
wherein the CRISPR complex includes a guide RNA and the CRISPR enzyme,
wherein the first nucleic acid is an on-target of the CRISPR complex,
wherein the second nucleic acid is an off-target of the CRISPR complex; identifying each cell,
whether or not the cell is dead, and
whether or not the first selection element is expressed,
whereby whether an off-target modification and an on-target modification occurs in the cell can be identified;
evaluating the specificity or the activity of the CRISPR enzyme based on the result of the identifying.

2. The method according to claim 1,
wherein the first selection element is a toxin gene, which is dependent on a species of the cell,
wherein,
the toxin gene is a ccdB gene when the cell is an E. coli (Escherichia coli);
the toxin gene is a HPRT (hypoxanthine phosphoribosyl transferase) gene when the cell is a mammalian cell; and
the toxin gene is a URA3 gene when the cell is yeast.

3. The method according to claim 1, wherein the CRISPR enzyme is a mutated CRISPR enzyme which is not naturally occurred.

4. The method according to claim 1, wherein the CRISPR enzyme is a Cas9.

5. The method according to claim 4,
wherein the Cas9 is selected from the group consists of: Streptococcus pyogenes Cas9 (SpCas9), Staphylococcus aureus Cas9 (SaCas9), Streptococcus thermophiles Cas9 (StCas9), Neisseria meningitides Cas9 (NmCas9), Campylobacter jejuni Cas9 (CjCas9) or orthologs thereof.

6. A cell comprising:
a first plasmid including,
a first nucleic acid, and
a first selection element being operably linked to the first nucleic acid,
wherein the first selection element is a gene selected from the group consists of a toxin gene, an antibiotic resistance gene, a gene encoding fluorescent protein, a tagging gene, a lacZ gene and a gene encoding luciferase; and
a genome including a second nucleic acid having a sequence homology less than 100% with the first nucleic acid;
wherein the first nucleic acid is an on-target of the CRISPR complex, wherein the second nucleic acid is an off-target of the CRISPR complex, wherein the second nucleic acid is exogenous, not being endogenous in the cell.

7. The cell according to claim 6,
wherein the first selection element is a toxin gene, which is dependent on a species of the cell,
wherein,
the toxin gene is a ccdB gene when the cell is an E. coli (Escherichia coli);
the toxin gene is a HPRT(hypoxanthine phosphoribosyl transferase) gene when the cell is a mammalian cell; and
the toxin gene is a URA3 gene when the cell is yeast.

8. A method for evaluating a specificity or an activity of a CRISPR enzyme, the method comprising:
providing at least one cell including a first plasmid and a second plasmid, wherein the first plasmid including:
a first nucleic acid; and
a first selection element being operably linked to the first nucleic acid,
wherein the first selection element is a gene selected from a group consist of a toxin gene, an antibiotic resistance gene, a gene encoding fluorescent protein, a tagging gene, a lacZ gene and a gene encoding luciferase,
wherein the second plasmid including:
a second nucleic acid having a sequence homology less than 100% with the first nucleic acid; and
a second selection element being operably linked to the second nucleic acid,
wherein the second selection element is a gene selected from a group consist of a toxin gene, an antibiotic resistance gene, a gene encoding fluorescent protein, a tagging gene, a lacZ gene and a gene encoding luciferase
contacting a CRISPR complex to the first nucleic acid and the second nucleic acid respectively,
wherein the CRISPR complex including a guide RNA and the CRISPR enzyme,
wherein the first nucleic acid is an on-target of the CRISPR complex,
wherein the second nucleic acid is an off-target of the CRISPR complex;
wherein the first selection element and the second selection element are different from each other;
identifying each cell,
whether or not the first selection element is expressed, and
whether or not the second selection element is expressed,
whereby whether an off-target modification and an on-target modification occurs in the cell can be identified;
evaluating the specificity or the activity of the CRISPR enzyme based on the result of the identifying.

9. The method according to claim 8,
wherein the first selection element is a toxin gene, which is dependent on a species of the cell,
wherein,
the toxin gene is a ccdB gene when the cell is an E. coli (Escherichia coli);
the toxin gene is a HPRT (hypoxanthine phosphoribosyl transferase) gene when the cell is a mammalian cell; and
the toxin gene is a URA3 gene when the cell is yeast.

10. The method according to claim 8,
wherein the CRISPR enzyme is a mutated CRISPR enzyme which is not naturally occurred.

11. The method according to claim 8,
wherein the CRISPR enzyme is a Cas9.

12. The method according to claim 11,
wherein the Cas9 is selected from the group consists of: Streptococcus pyogenes Cas9 (SpCas9), Staphylococcus aureus Cas9 (SaCas9), Streptococcus thermophiles Cas9 (StCas9), Neisseria meningitides Cas9 (NmCas9), Campylobacter jejuni Cas9 (CjCas9) or orthologs thereof.

13. A cell comprising:
a first plasmid including,
a first nucleic acid, and
a first selection element being operably linked to the first nucleic acid,
wherein the first selection element is a gene selected from a group consist of a toxin gene, an antibiotic resistance gene, a gene encoding fluorescent protein, a tagging gene, a lacZ gene and a gene encoding luciferase; and
a second plasmid including,
a second nucleic acid having a sequence homology less than 100% with the first nucleic acid, and
a second selection element being operably linked to the second nucleic acid,
wherein the second selection element is a gene selected from a group consist of a toxin gene, an antibiotic resistance gene, a gene encoding fluorescent protein, a tagging gene, a lacZ gene and a gene encoding luciferase;
wherein the first nucleic acid is an on-target of the CRISPR complex,
wherein the second nucleic acid is an off-target of the CRISPR complex,
wherein the first selection element and the second selection element are different from each other.

14. The cell according to claim 13,
wherein the first selection element is a toxin gene, which is dependent on a species of the cell,
wherein, the toxin gene is a ccdB gene when the cell is an E. coli (Escherichia coli); the toxin gene is a HPRT (hypoxanthine phosphoribosyl transferase) gene when the cell is a mammalian cell; and
the toxin gene is a URA3 gene when the cell is yeast.

15. A kit for evaluating a specificity or an activity of a CRISPR enzyme including the cell according to claim 6 or 13.

## Patentansprüche

1. Verfahren zum Bewerten einer Spezifität oder Aktivität eines CRISPR-Enzyms, wobei das Verfahren umfasst:
Bereitstellen wenigstens einer Zelle, die ein erstes Plasmid und ein Genom umfasst;
wobei das erste Plasmid umfasst:
eine erste Nucleinsäure und
ein erstes Selektionselement, das funktionell mit der ersten Nucleinsäure verknüpft ist;
wobei das erste Selektionselement ein Gen ist, das aus der Gruppe ausgewählt ist, die aus einem Toxin-Gen, einem Antibiotikaresistenz-Gen, einem Gen, das ein fluoreszierendes Protein codiert, einem Marker-Gen, einem lacZ-Gen und einem Gen, das Luciferase codiert, besteht;
wobei das Genom eine zweite Nucleinsäure umfasst, die eine Sequenzhomologie von weniger als 100% mit der ersten Nucleinsäure aufweist;
In-Kontakt-Bringen eines CRISPR-Komplexes mit der ersten Nucleinsäure und der zweiten Nucleinsäure;
wobei der CRISPR-Komplex eine Führungs-RNA und das CRISPR-Enzym umfasst;
wobei die erste Nucleinsäure ein On-Target des CRISPR-Komplexes ist;
wobei die zweite Nucleinsäure ein Off-Target des CRISPR-Komplexes ist;
Identifizieren bei jeder Zelle:
ob die Zelle tot ist oder nicht; und
ob das erste Selektionselement exprimiert wird oder nicht;
wodurch identifiziert werden kann, ob in der Zelle eine Off-Target-Modifikation und eine On-Target-Modifikation stattfindet;
Bewerten der Spezifität oder Aktivität des CRISPR-Enzyms auf der Basis des Ergebnisses der Identifizierung.

2. Verfahren gemäß Anspruch 1,
wobei das erste Selektionselement ein Toxin-Gen ist, das von der Spezies der Zelle abhängt;
wobei
das Toxin-Gen ein ccdB-Gen ist, wenn die Zelle ein *E. coli (Escherichia coli)* ist;
das Toxin-Gen ein HPRT(Hypoxanthin-Phosphoribosyl-Transferase)-Gen ist, wenn die Zelle eine Säugerzelle ist; und
das Toxin-Gen ein URA3-Gen ist, wenn die Zelle eine Hefe ist.

3. Verfahren gemäß Anspruch 1, wobei das CRISPR-Enzym ein mutiertes CRISPR-Enzym ist, das nicht natürlich vorkommend ist.

4. Verfahren gemäß Anspruch 1, wobei das CRISPR-Enzym ein Cas9 ist.

5. Verfahren gemäß Anspruch 4, wobei das Cas9 aus der Gruppe ausgewählt ist, die aus *Streptococcus pyogenes* Cas9 (SpCas9), *Staphylococcus aureus* Cas9 (SaCas9), *Streptococcus thermophiles* Cas9 (StCas9), *Neisseria meningitides* Cas9 (NmCas9), *Campylobacter jejuni* Cas9 (CjCas9) oder Orthologen davon besteht.

6. Zelle, umfassend:
ein erstes Plasmid, umfassend:
eine erste Nucleinsäure und
ein erstes Selektionselement, das funktionell mit der ersten Nucleinsäure verknüpft ist;
wobei das erste Selektionselement ein Gen ist, das aus der Gruppe ausgewählt ist, die aus einem Toxin-Gen, einem Antibiotikaresistenz-Gen, einem Gen, das ein fluoreszierendes Protein codiert, einem Marker-Gen, einem lacZ-Gen und einem Gen, das Luciferase codiert, besteht; und
ein Genom, das eine zweite Nucleinsäure umfasst, die eine Sequenzhomologie von weniger als 100% mit der ersten Nucleinsäure aufweist;
wobei die erste Nucleinsäure ein On-Target des CRISPR-Komplexes ist, wobei die zweite Nucleinsäure ein Off-Target des CRISPR-Komplexes ist, wobei die zweite Nucleinsäure exogen ist und in der Zelle nicht endogen ist.

7. Zelle gemäß Anspruch 6,
wobei das erste Selektionselement ein Toxin-Gen ist, das von der Spezies der Zelle abhängt;
wobei
das Toxin-Gen ein ccdB-Gen ist, wenn die Zelle ein *E. coli (Escherichia coli)* ist;
das Toxin-Gen ein HPRT(Hypoxanthin-Phosphoribosyl-Transferase)-Gen ist, wenn die Zelle eine Säugerzelle ist; und
das Toxin-Gen ein URA3-Gen ist, wenn die Zelle eine Hefe ist.

8. Verfahren zum Bewerten einer Spezifität oder Aktivität eines CRISPR-Enzyms, wobei das Verfahren umfasst:
Bereitstellen wenigstens einer Zelle, die ein erstes Plasmid und ein zweites Plasmid umfasst;
wobei das erste Plasmid umfasst:
eine erste Nucleinsäure und
ein erstes Selektionselement, das funktionell mit der ersten Nucleinsäure verknüpft ist;
wobei das erste Selektionselement ein Gen ist, das aus der Gruppe ausgewählt ist, die aus einem Toxin-Gen, einem Antibiotikaresistenz-Gen, einem Gen, das ein fluoreszierendes Protein codiert, einem Marker-Gen, einem lacZ-Gen und einem Gen, das Luciferase codiert, besteht;
wobei das zweite Plasmid umfasst:
eine zweite Nucleinsäure, die eine Sequenzhomologie von weniger als 100% mit der ersten Nucleinsäure aufweist; und
ein zweites Selektionselement, das funktionell mit der zweiten Nucleinsäure verknüpft ist;
wobei das zweite Selektionselement ein Gen ist, das aus der Gruppe ausgewählt ist, die aus einem Toxin-Gen, einem Antibiotikaresistenz-Gen, einem Gen, das ein fluoreszierendes Protein codiert, einem Marker-Gen, einem lacZ-Gen und einem Gen, das Luciferase codiert, besteht;
In-Kontakt-Bringen eines CRISPR-Komplexes mit der ersten Nucleinsäure und der zweiten Nucleinsäure;
wobei der CRISPR-Komplex eine Führungs-RNA und das CRISPR-Enzym umfasst;
wobei die erste Nucleinsäure ein On-Target des CRISPR-Komplexes ist;
wobei die zweite Nucleinsäure ein Off-Target des CRISPR-Komplexes ist;
wobei das erste Selektionselement und das zweite Selektionselement voneinander verschieden sind;
Identifizieren bei jeder Zelle:
ob das erste Selektionselement exprimiert wird oder nicht; und
ob das zweite Selektionselement exprimiert wird oder nicht;
wodurch identifiziert werden kann, ob in der Zelle eine Off-Target-Modifikation und eine On-Target-Modifikation stattfindet;
Bewerten der Spezifität oder Aktivität des CRISPR-Enzyms auf der Basis des Ergebnisses der Identifizierung.

9. Verfahren gemäß Anspruch 8,
wobei das erste Selektionselement ein Toxin-Gen ist, das von der Spezies der Zelle abhängt;
wobei
das Toxin-Gen ein ccdB-Gen ist, wenn die Zelle ein *E. coli (Escherichia coli)* ist;
das Toxin-Gen ein HPRT(Hypoxanthin-Phosphoribosyl-Transferase)-Gen ist, wenn die Zelle eine Säugerzelle ist; und
das Toxin-Gen ein URA3-Gen ist, wenn die Zelle eine Hefe ist.

10. Verfahren gemäß Anspruch 8, wobei das CRISPR-Enzym ein mutiertes CRISPR-Enzym ist, das nicht natürlich vorkommend ist.

11. Verfahren gemäß Anspruch 8, wobei das CRISPR-Enzym ein Cas9 ist.

12. Verfahren gemäß Anspruch 11, wobei das Cas9 aus der Gruppe ausgewählt ist, die aus *Streptococcus pyogenes* Cas9 (SpCas9), *Staphylococcus aureus* Cas9 (SaCas9), *Streptococcus thermophiles* Cas9 (StCas9), *Neisseria meningitides* Cas9 (NmCas9), *Campylobacter jejuni* Cas9 (CjCas9) oder Orthologen davon besteht.

13. Zelle, umfassend:
ein erstes Plasmid, umfassend:
eine erste Nucleinsäure und
ein erstes Selektionselement, das funktionell mit der ersten Nucleinsäure verknüpft ist;
wobei das erste Selektionselement ein Gen ist, das aus der Gruppe ausgewählt ist, die aus einem Toxin-Gen, einem Antibiotikaresistenz-Gen, einem Gen, das ein fluoreszierendes Protein codiert, einem Marker-Gen, einem lacZ-Gen und einem Gen, das Luciferase codiert, besteht; und
ein zweites Plasmid, umfassend:
eine zweite Nucleinsäure, die eine Sequenzhomologie von weniger als 100% mit der ersten Nucleinsäure aufweist; und
ein zweites Selektionselement, das funktionell mit der zweiten Nucleinsäure verknüpft ist;
wobei das zweite Selektionselement ein Gen ist, das aus der Gruppe ausgewählt ist, die aus einem Toxin-Gen, einem Antibiotikaresistenz-Gen, einem Gen, das ein fluoreszierendes Protein codiert, einem Marker-Gen, einem lacZ-Gen und einem Gen, das Luciferase codiert, besteht;
wobei die erste Nucleinsäure ein On-Target des CRISPR-Komplexes ist;
wobei die zweite Nucleinsäure ein Off-Target des CRISPR-Komplexes ist;
wobei das erste Selektionselement und das zweite Selektionselement voneinander verschieden sind.

14. Zelle gemäß Anspruch 13,
wobei das erste Selektionselement ein Toxin-Gen ist, das von der Spezies der Zelle abhängt;
wobei
das Toxin-Gen ein ccdB-Gen ist, wenn die Zelle ein *E. coli* (*Escherichia coli*) ist;
das Toxin-Gen ein HPRT(Hypoxanthin-Phosphoribosyl-Transferase)-Gen ist, wenn die Zelle eine Säugerzelle ist; und
das Toxin-Gen ein URA3-Gen ist, wenn die Zelle eine Hefe ist.

15. Kit zum Bewerten einer Spezifität oder Aktivität eines CRISPR-Enzyms, der die Zelle gemäß Anspruch 6 oder 13 umfasst.

## Revendications

1. Procédé pour évaluer une spécificité ou une activité de l'enzyme CRISPR, ledit procédé comprenant les étapes consistant à :
procurer au moins une cellule comprenant un premier plasmide et un génome,
dans lequel le premier plasmide comprend
un premier acide nucléique, et
un premier élément de sélection étant opérationnellement lié au premier acide nucléique,
dans lequel le premier élément de sélection est un gène choisi dans le groupe consistant en un gène de toxine, un gène de résistance aux antibiotiques, un gène codant pour une protéine fluorescente, un gène marqueur, un gène lacZ, et un gène codant pour luciférase,
dans lequel ledit génome comprend un second acide nucléique ayant une homologie de séquence de moins de 100 % avec le premier acide nucléique,
contacter un complexe CRISPR au premier acide nucléique et au second acide nucléique,
où ledit complexe CRISPR comprend un ARN guide et ladite enzyme CRISPR,
dans lequel le premier acide nucléique est un site sur cible du complexe CRISPR,
dans lequel le second acide nucléique est un site hors cible du complexe CRISPR,
identifier pour chaque cellule
si la cellule est morte ou non, et
si le premier élément de sélection est exprimé ou non,
qui permet d'identifier si une modification hors cible et une modification sur cible se passe dans la cellule,
évaluer la spécificité ou l'activité de l'enzyme CRISPR sur la base du résultat de l'identification.

2. Procédé selon la revendication 1,
dans lequel le premier élément de sélection est un gène de toxine, qui dépend sur l'espèce de la cellule,
dans lequel
ledit gène de toxine est un gène ccdB si la cellule est une *E. coli* (*Escherichia coli*),
ledit gène de toxine est un gène de HPRT (hypoxanthine phosphoribosyl transférase) si la cellule est une cellule de mammifère, et
ledit gène de toxine est un gène URA3 si la cellule est une levure.

3. Procédé selon la revendication 1, dans lequel ladite enzyme CRISPR est une enzyme CRISPR mutée qui n'est pas naturellement présente.

4. Procédé selon la revendication 1, dans lequel ladite enzyme CRISPR est une Cas9.

5. Procédé selon la revendication 4, dans lequel ladite Cas9 est choisie dans le groupe consistant en *Streptococcus pyogenes* Cas9 (SpCas9), *Staphylococcus aureus* Cas9 (SaCas9), *Streptococcus thermophiles* Cas9 (StCas9), *Neisseria meningitides* Cas9 (NmCas9), *Campylobacter jejuni* Cas9 (CjCas9) ou des orthologues de ceux-ci.

6. Cellule, comprenant :
un premier plasmide, comprenant
un premier acide nucléique, et
un premier élément de sélection étant opérationnellement lié au premier acide nucléique,
dans lequel le premier élément de sélection est un gène choisi dans le groupe consistant en un gène de toxine, un gène de résistance aux antibiotiques, un gène codant pour une protéine fluorescente, un gène marqueur, un gène lacZ, et un gène codant pour luciférase, et
un génome comprenant un second acide nucléique ayant une homologie de séquence de moins de 100 % avec le premier acide nucléique,
dans lequel le premier acide nucléique est un site sur cible du complexe CRISPR, dans lequel le second acide nucléique est un site hors cible du complexe CRISPR, dans lequel le second acide nucléique est exogène, n'étant pas endogène dans la cellule.

7. Cellule selon la revendication 6,
dans lequel le premier élément de sélection est un gène de toxine, qui dépend sur l'espèce de la cellule,
dans lequel
ledit gène de toxine est un gène ccdB si la cellule est une *E. coli* (*Escherichia coli*),
ledit gène de toxine est un gène de HPRT (hypoxanthine phosphoribosyl transférase) si la cellule est une cellule de mammifère, et
ledit gène de toxine est un gène URA3 si la cellule est une levure.

8. Procédé pour évaluer une spécificité ou une activité de l'enzyme CRISPR, ledit procédé comprenant les étapes consistant à :
procurer au moins une cellule comprenant un premier plasmide et un second plasmide, dans lequel le premier plasmide comprend
un premier acide nucléique, et
un premier élément de sélection étant opérationnellement lié au premier acide nucléique,
dans lequel le premier élément de sélection est un gène choisi dans le groupe consistant en un gène de toxine, un gène de résistance aux antibiotiques, un gène codant pour une protéine fluorescente, un gène marqueur, un gène lacZ, et un gène codant pour luciférase,
dans lequel le second plasmide comprend
un second acide nucléique ayant une homologie de séquence de moins de 100 % avec le premier acide nucléique, et
un second élément de sélection étant opérationnellement lié au second acide nucléique,
dans lequel le second élément de sélection est un gène choisi dans le groupe consistant en un gène de toxine, un gène de résistance aux antibiotiques, un gène codant pour une protéine fluorescente, un gène marqueur, un gène lacZ, et un gène codant pour luciférase,
contacter un complexe CRISPR au premier acide nucléique et au second acide nucléique,
où ledit complexe CRISPR comprend un ARN guide et ladite enzyme CRISPR,
dans lequel le premier acide nucléique est un site sur cible du complexe CRISPR,
dans lequel le second acide nucléique est un site hors cible du complexe CRISPR,
dans lequel le premier élément de sélection et le second élément de sélection sont différents l'un de l'autre,
identifier pour chaque cellule
si le premier élément de sélection est exprimé ou non, et
si le second élément de sélection est exprimé ou non,
qui permet d'identifier si une modification hors cible et une modification sur cible se passe dans la cellule,
évaluer la spécificité ou l'activité de l'enzyme CRISPR sur la base du résultat de l'identification.

9. Procédé selon la revendication 8,
dans lequel le premier élément de sélection est un gène de toxine, qui dépend sur l'espèce de la cellule,
dans lequel
ledit gène de toxine est un gène ccdB si la cellule est une *E. coli* (*Escherichia coli*),
ledit gène de toxine est un gène de HPRT (hypoxanthine phosphoribosyl transférase) si la cellule est une cellule de mammifère, et
ledit gène de toxine est un gène URA3 si la cellule est une levure.

10. Procédé selon la revendication 8, dans lequel ladite enzyme CRISPR est une enzyme CRISPR mutée qui n'est pas naturellement présente.

11. Procédé selon la revendication 8, dans lequel ladite enzyme CRISPR est une Cas9.

12. Procédé selon la revendication 11, dans lequel ladite Cas9 est choisie dans le groupe consistant en *Streptococcus pyogenes* Cas9 (SpCas9), *Staphylococcus aureus* Cas9 (SaCas9), *Streptococcus thermophiles* Cas9 (StCas9), *Neisseria meningitides* Cas9 (NmCas9), *Campylobacter jejuni* Cas9 (CjCas9) ou des orthologues de ceux-ci.

13. Cellule, comprenant :
un premier plasmide, comprenant
un premier acide nucléique, et
un premier élément de sélection étant opérationnellement lié au premier acide nucléique,
dans lequel le premier élément de sélection est un gène choisi dans le groupe consistant en un gène de toxine, un gène de résistance aux antibiotiques, un gène codant pour une protéine fluorescente, un gène marqueur, un gène lacZ, et un gène codant pour luciférase, et
un second plasmide, comprenant
un second acide nucléique ayant une homologie de séquence de moins de 100 % avec le premier acide nucléique, et
un second élément de sélection étant opérationnellement lié au second acide nucléique,
dans lequel le second élément de sélection est un gène choisi dans le groupe consistant en un gène de toxine, un gène de résistance aux antibiotiques, un gène codant pour une protéine fluorescente, un gène marqueur, un gène lacZ, et un gène codant pour luciférase,
dans lequel le premier acide nucléique est un site sur cible du complexe CRISPR,
dans lequel le second acide nucléique est un site hors cible du complexe CRISPR,
dans lequel le premier élément de sélection et le second élément de sélection sont différents l'un de l'autre.

14. Cellule selon la revendication 13,
dans lequel le premier élément de sélection est un gène de toxine, qui dépend sur l'espèce de la cellule,
dans lequel
ledit gène de toxine est un gène ccdB si la cellule est une *E. coli* (*Escherichia coli*),
ledit gène de toxine est un gène de HPRT (hypoxanthine phosphoribosyl transférase) si la cellule est une cellule de mammifère, et
ledit gène de toxine est un gène URA3 si la cellule est une levure.

15. Trousse pour évaluer une spécificité ou une activité de l'enzyme CRISPR, comprenant la cellule selon la revendication 6 ou 13.


<110> TOOLGEN INCORPORATED
<120> A Method for screening target specific Nuclease using multi-target system of on-target and off-target, and the Use thereof
<130> OPP17-033-PCT
<150> PCT/US 62/350,282
   <151> 2016-06-15
<160> 10
<170> KoPatentIn 3.0
<210> 1
   <211> 23
   <212> DNA
   <213> Escherichia coli
<400> 1
   catcaacatc gaatacatga nag 23
<210> 2
   <211> 20
   <212> RNA
   <213> Artificial Sequence
<220>
   <223> single guide RNA sequence
<400> 2
   catcaacatc gaatacatga 20
<210> 3
   <211> 23
   <212> DNA
   <213> Artificial Sequence
<220>
   <223> target site sequence
<400> 3
   catcaacatc gaatacatga tgg 23
<210> 4
   <211> 28
   <212> DNA
   <213> Artificial Sequence
<220>
   <223> DNA sequence
<400> 4
   ctagatgaga ccggatccgg tctcctgg 28
<210> 5
   <211> 25
   <212> DNA
   <213> Artificial Sequence
<220>
   <223> DNA sequence
<400> 5
   ctagatgaga ccggatccgg tctcc 25
<210> 6
   <211> 23
   <212> DNA
   <213> Artificial Sequence
<220>
   <223> target site sequence
<400> 6
   gagtccgagc agaagaagaa ggg 23
<210> 7
   <211> 20
   <212> RNA
   <213> Artificial Sequence
<220>
   <223> single guide RNA sequence
<400> 7
   gagtccgagc agaaagagaa 20
<210> 8
   <211> 20
   <212> RNA
   <213> Artificial Sequence
<220>
   <223> single guide RNA sequence
<400> 8
   gaacccgagc agaagaagaa 20
<210> 9
   <211> 20
   <212> RNA
   <213> Artificial Sequence
<220>
   <223> single guide RNA sequence
<400> 9
   gagtccgagc agaggaagaa 20
<210> 10
   <211> 20
   <212> RNA
   <213> Artificial Sequence
<220>
   <223> single guide RNA sequence
<400> 10
   gagcccgagc agaagaagaa 20